# EUROPEAN PATENT SPECIFICATION

(11) **EP 0 824 317 B1**
(45) Date of publication and mention of the grant of the patent: **13.10.2004**
(21) Application number: 96906949.1
(22) Date of filing: 26.03.1996
(51) Int. Cl.: A01N 43/82, C07D 285/06

(54) **AN AGRICULTURAL AND HORTICULTURAL DISEASE CONTROLLER AND A METHOD FOR CONTROLLING THE DISEASES**
SCHÄDLINGSBEKÄMPFUNGSMITTEL FÜR LANDWIRTSCHAFT UND GARTENBAU UND VERFAHREN ZUR BEKÄMPFUNG VON SCHÄDLINGEN
AGENT DE LUTTE CONTRE LES MALADIES DANS LES DOMAINES DE L'AGRICULTURE ET DE L'HORTICULTURE ET PROCEDE DE LUTTE CONTRE CES MALADIES

(30) Priority: 31.03.1995 JP 9988095
(43) Date of publication of application: 25.02.1998
(60) Divisional application: 04001677.6
(73) Proprietor: Nihon Nohyaku Co., Ltd., Tokyo 103 (JP)
(72) Inventor: KURODA, Kiyoshi, Sakura-shi, Chiba 285 (JP); UCHIKUROHANE, Toru, Hashimoto-shi, Wakayama 648 (JP); TAJIMA, Sokichi, Osaka-shi, Osaka 558 (JP); TSUBATA, Kenji, Kawachinagano-shi, Osaka 586 (JP)
(74) Representative: Grünecker, Kinkeldey, Stockmair & Schwanhäusser Anwaltssozietät
(86) International application number: PCT/JP1996/000781
(87) International publication number: WO 1996/029871

(56) References cited:
- EP-A- 0 046 497
- FR-A- 2 395 263
- FR-A- 2 451 371
- FR-A- 2 453 165

## Description

The present invention relates to the use of a composition which comprises an inert carrier and an active amount of a 1,2,3-thiadiazole derivative represented by the general formula (I), or a salt thereof for controlling agricultural and horticultural diseases: 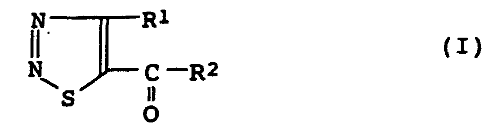 wherein R¹ is ① a hydrogen atom, ② a (C₁-C₁₂)alkyl group, ③ a halo(C₁-C₁₂)alkyl group, ④ a (C₂-C₁₂)-alkenyl group, ⑤ a halo(C₂-C₁₂)alkenyl group, ⑥ a (C₂-C₁₂)alkynyl group, ⑦ a halo(C₂-C₁₂)alkynyl group, ⑧ a (C₃-C₆)cycloalkyl group, ⑨ an unsubstituted phenyl group, 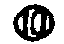 a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, 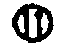 a group represented by the formula: 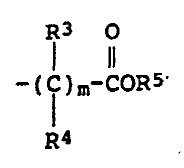 (wherein R³ and R⁴, which may be the same or different, are hydrogen atoms, (C₁-C₁₂)alkyl groups or halo(C₁-C₁₂)alkyl groups, m is zero or an integer of 1 to 6, and R⁵ is a hydrogen atom; a (C₁-C₁₂)alkyl group; a halo(C₁-C₁₂)alkyl group; a (C₂-C₁₂)alkenyl group; a (C₂-C₁₂)alkynyl group; an unsubstituted phenyl group; a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted phenyl(C₁-C₆)alkyl group; or a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups), 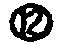 a group represented by the formula: 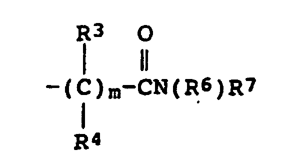 (wherein R³, R⁴ and m are as defined above, and R⁶ and R⁷, which may be the same or different, are hydrogen atoms; (C₁-C₁₂)alkyl groups; halo(C₁-C₁₂)alkyl groups; (C₂-C₁₂)alkenyl groups; (C₂-C₁₂)alkynyl groups; (C₁-C₁₂)alkoxy(C₁-C₆)alkyl groups; (C₁-C₁₂)alkylthio(C₁-C₆)alkyl groups; cyano(C₁-C₁₂)alkyl groups; unsubstituted phenyl groups; substituted phenyl groups having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; unsubstituted phenyl(C₁-C₆)alkyl groups; or substituted phenyl(C₁-C₆)alkyl groups having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; R⁶ and R⁷ being able to be taken together to represent a (C₄-C₆)alkylene group which may contain an oxygen atom between adjacent carbon atoms of the carbon chain), 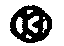 a group represented by the formula: 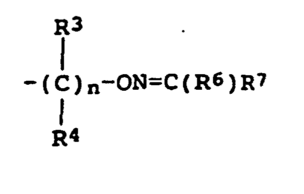 (wherein R³, R⁴, R⁶ and R⁷ are as defined above, and n is an integer of 1 to 6), or 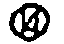 a group represented by the formula: 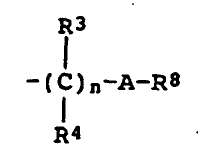 [wherein R³, R⁴ and n are as defined above, A is

-O- ,

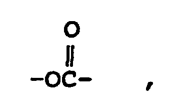

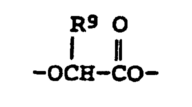

(wherein R⁹ is a hydrogen atom, a (C₁-C₁₂)alkyl group or a halo(C₁-C₁₂)alkyl group),

-S- ,

-SO- ,

-SO₂- ,

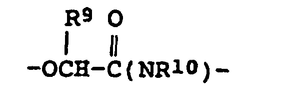

{wherein R⁹ is as defined above, and R¹⁰ is a hydrogen atom, a (C₁-C₁₂)alkyl group or a halo(C₁-C₁₂)alkyl group, R⁹ and R¹⁰ being able to be taken together to represent a (C₄-C₆)alkylene group which may contain, between adjacent carbon atoms of the carbon chain, an oxygen atom, a sulfur atom or

N-R¹¹

(wherein R¹¹ is a hydrogen atom, a (C₁-C₁₂)alkyl group or a halo(C₁-C₁₂)alkyl group)}, or

-N(R¹⁰)-

(wherein R¹⁰ is as defined above), and R⁸ is a hydrogen atom; a (C₁-C₁₂)alkyl group; a halo(C₁-C₁₂)alkyl group; a (C₂-C₁₂)alkenyl group; a (C₂-C₁₂)alkynyl group; a (C₁-C₁₂)alkoxy(C₁-C₁₂)alkyl group; a (C₁-C₁₂)alkylthio(C₁-C₁₂)alkyl group; a cyano(C₁-C₁₂)alkyl group; an unsubstituted phenyl group; a substituted phenyl group having one or more substituents which may be the same or different and are selected from the group consisting of halogen atoms, nitro groups cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted phenyl(C₁-C₆)-alkyl group; a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted 5- or 6-membered heterocyclic ring having one or more heteroatoms which may be the same or different and are selected from the group consisting of oxygen atom, sulfur atom and nitrogen atom; or a substituted 5- or 6-membered heterocyclic ring having one or more heteroatoms which may be the same or different and are selected from the group consisting of oxygen atom, sulfur atom and nitrogen atom, said substituted heterocyclic ring having one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups], and R² is a group represented by the formula:

- X-R¹²

[wherein X is an oxygen atom, and R¹²
is ① a hydrogen atom, ② a (C₁-C₁₂)alkyl group, ③ a halo(C₁-C₁₂)alkyl group, ④ a (C₂-C₂₀)alkenyl group, ⑤ a halo(C₂-C₂₀)alkenyl group, ⑥ a (C₂-C₁₂)alkynyl group, ⑦ a halo(C₂-C₁₂)alkynyl group, ⑧ a hydroxy(C₁-C₆)alkyl group, ⑨ a (C₁-C₁₂)alkoxy(C₁-C₁₂)alkyl group, 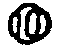 a (C₁-C₁₂)alkylthio(C₁-C₁₂)alkyl group, 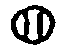 a (C₃-C₆)cycloalkyl group,
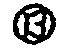 an unsubstituted phenyl group, 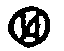 a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkylthio groups, halo(C₁-C₆)alkylthio groups, (C₂-C₆)alkenyl 10 groups, (C₂-C₆)alkynyl groups, and (C₁-C₆)alkoxycarbonyl(C₁-C₆)alkyloxy groups 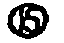 an unsubstituted phenyl(C₁-C₆)alkyl group, 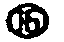 a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)ailkynyl groups, 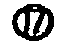 a diphenyl(C₁-C₆)alkyl group, 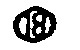 a phenoxy(C₁-C₆)alkyl group, 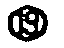 a group represented by the formula:

-B-N(R¹³)R¹⁴

{wherein B is a (C₁-C₆)alkylene group which may be substituted by a (C₁-C₆)alkyl group,
and R¹³ and R¹⁴, which may be the same or different, are hydrogen atoms; formyl groups; (C₁-C₁₂)alkyl groups; (C₂-C₁₂)alkenyl groups; (C₂-C₁₂)alkynyl groups; (C₁-C₆) alkylcarbonyl groups; unsubstituted phenyl groups; substituted phenyl groups having one or more substituents which may be the same or different and are selected from the group consisting of hydrogen atom, halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; phenylcarbonyl groups; unsubstituted 1,2,3-thiadiazol-5-yl-carbonyl groups; or substituted 1,2,3-thiadiazol-5-yl-carbonyl groups having a halogen atom or a (C₁-C₆)alkyl group as the substituent; R¹³ and R¹⁴ being able to be taken together to represent a (C₄-C₅)alkylene group which may contain, between adjacent carbon atoms of the carbon chain, an oxygen atom, a sulfur atom or

N-R¹¹

(wherein R¹¹ is as defined above)}, 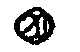 a group represented by the formula:

-C(R¹⁵)=C(R¹⁶)-R¹⁷

{wherein R¹⁵ is a hydrogen atom or a (C₁-C₆)alkyl group, R¹⁶ is a hydrogen atom, a halogen atom or a (C₁-C₆)alkyl group, and R¹⁷ is a nitro group, a cyano group, a (C₁-C₆)alkylcarbonyl group, a (C₁-C₆)alkoxycarbonyl group, a phenylcarbonyl group, or a substituted aminocarbonyl group having one or more substituents which may be the same or different and are selected from the group consisting of hydrogen atom, (C₁-C₁₂)alkyl groups, unsubstituted phenyl group, and substituted phenyl groups having one or more substituents which may be the same or different and are selected from the group consisting of halogen atoms, cyano group, nitro group, (C₁-C₆)alkyl groups and (C₁-C₆)alkoxy groups, R¹⁵ and R¹⁷ being able to be taken together to represent a (C₃-C₆)alkylene group which may be substituted by one or more (C₁-C₆)alkyl group and/or an oxo group), 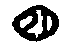 a group represented by the formula: 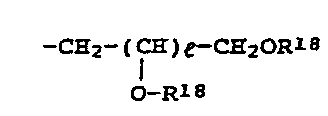 (wherein two R¹⁸'s, which may be the same or different, are hydrogen atoms, (C₁-C₆)alkylcarbonyl groups, phenylcarbonyl groups, unsubstituted 1,2,3-thiadiazol-5-yl-carbonyl groups, or substituted 1,2,3-thiadiazol-5-yl-carbonyl groups having a halogen atom or a (C₁-C₆)-alkyl group as the substituent, and ℓ is zero or an integer of 1 to 12), 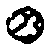 tri(C₁-C₆)alkylsilyl(C₁-C₆)alkyl groups, or 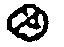 1,2,3-thiadiazol-5-yl-carbonyloxy(C₁-C₁₂)alkyl groups having on the ring a halogen atom or (C₁-C₆)alkyl group as the substituent].

Preferably, in the 1,2,3-thiadiazole derivative of general formula (I) R¹ is ① a hydrogen atom, ② a (C₁-C₁₂)alkyl group, ③ a halo(C₁-C₁₂)alkyl group, ④ a (C₂-C₁₂)alkenyl group, ⑤ a halo(C₂-C₁₂)alkenyl group, ⑥ a (C₂-C₁₂)alkynyl group, ⑦ a halo(C₂-C₁₂)alkynyl group, ⑧ a (C₃-C₆)cycloalkyl group, ⑨ an unsubstituted phenyl group, or 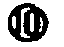 a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, and R² is a group represented by the formula:

-X-R¹²

[wherein X is an oxygen atom and R¹²
is ① a hydrogen atom, ② a (C₁-C₁₂)alkyl group, ③ a halo(C₁-C₁₂)alkyl group, ④ a (C₂-C₂₀)alkenyl group, ⑤ a halo(C₂-C₂₀)alkenyl group, ⑥ a (C₂-C₁₂)alkynyl group, ⑦ a halo(C₂-C₁₂)alkynyl group, ⑧ a hydroxy(C₁-C₆)alkyl group, ⑨ a (C₁-C₁₂)alkoxy(C₁-C₁₂)alkyl group, 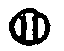 a (C₃-C₆)cycloalkyl group, 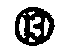 an unsubstituted phenyl group, 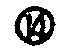 a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, and (C₁-C₆)alkoxycarbonyl(C₁-C₆)alkyloxy groups, 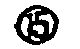 an unsubstituted phenyl(C₁-C₆)alkyl group, 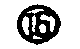 a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, and halo(C₁-C₆)alkoxy groups, 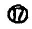 a diphenyl(C₁-C₆)alkyl group, 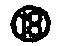 a phenoxy(C₁-C₆)alkyl group, 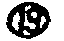 a group represented by the formula:

-B-N(R¹³)R¹⁴

{wherein B is a (C₁-C₆)alkylene group which may be substituted by a (C₁-C₆)alkyl group,
and R¹³ and R¹⁴, which may be the same or different, are hydrogen atoms; formyl groups; (C₁-C₁₂)alkyl groups; (C₂-C₁₂)alkenyl groups; (C₂-C₁₂)alkynyl groups; (C₁-C₁₂)alkylcarbonyl groups; unsubstituted phenyl groups; substituted phenyl groups having one or more substituents which may be the same or different and are selected from the group consisting of hydrogen atom, halogen atoms, nitro group, cyano group, (C₁-C₆)alkyl groups, halo(C₁-C₆)alkyl groups, (C₁-C₆)alkoxy groups, and halo(C₁-C₆)alkoxy groups; phenylcarbonyl groups; unsubstituted 1,2,3-thiadiazol-5-yl-carbonyl groups; or substituted 1,2,3-thiadiazol-5-yl-carbonyl groups having a halogen atom or a (C₁-C₆)alkyl group as the substituent; 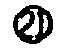 a group represented by the formula: 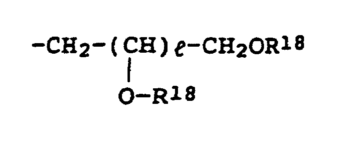 (wherein two R¹⁸'s, which may be the same or different, are hydrogen atoms, (C₁-C₆)alkylcarbonyl groups, phenylcarbonyl groups, unsubstituted 1,2,3-thiadiazol-5-yl-carbonyl groups, or substituted 1,2,3-thiadiazol-5-yl-carbonyl groups having a halogen atom or a (C₁-C₆)-alkyl group as the substituent, and ℓ is zero or an integer of 1 to 12), or 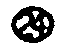 1,2,3-thiadiazol-5-yl-carbonyloxy(C₁-C₆)alkyl groups having on the ring a halogen atom or (C₁-C₆)alkyl group as the substituent].

The present invention further relates to a method for controlling agricultural and horticultural diseases which comprises applying the composition for controlling agricultural and horticultural diseases as defined above in a dosage of 0.1 to 10 kg (in terms of the active ingredient of the disease controller) per 1000 m² (10 ares) for protecting useful crop against the diseases.

In addition, the present invention relates to the use of a 1,2,3-thiadiazole derivative represented by the general formula (I), or a salt thereof 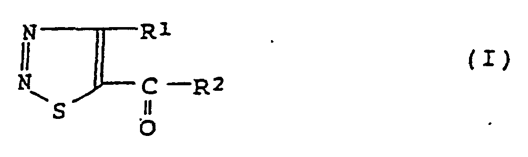 wherein R¹ and R² are as defined above for controlling agricultural and horticultural diseases.

FR-A-2453165 and FR-A- 2451371 disclose 1,2,3-thiadiazole-5-carboxylic acid derivatives having herbicidal, growth regulating, defoliating and fungicidal activities.

In EP-A- 0046497 1,2,3-thiadiazole derivatives are disclosed which correspond to the 1,2,3-thiadiazole derivative of formula (I) according to the present invention, except that the group R² is an N-phenylalanine derivative.

Japanese Patent Unexamined Publication No. 54-9272 discloses 1,2,3-thiadiazole-5-carboxylic acid derivatives, a process for production thereof and an agent containing the derivative and having herbicidal and growth-regulating effects.

The present inventors earnestly investigated for developing a novel agricultural and horticultural disease controller and consequently found that 1,2,3-thiadiazole derivatives or salts thereof, which include some of the compounds disclosed in USP 4177054, are useful for herbicide and growth segulater, whereby the present invention has been accomplished.

### Best Mode for Carrying Out the Invention

In the definition of the substituents of the 1,2,3-thiadiazole derivative of the general formula (I) used as the active ingredient of the agricultural and horticultural disease controller of the present invention, the halogen atom includes chlorine atom, bromine atom, iodine atom and fluorine atom. The term "(C₁-C₁₂)alkyl group" means a linear or branched alkyl group of 1 to 12 carbon atoms, such as methyl, ethyl, n-propyl, i-propyl, n-butyl, i-butyl, s-butyl, t-butyl, n-pentyl, n-hexyl, n-heptyl, n-octyl, n-nonyl, n-decyl, n-undecyl, n-dodecyl or the like. The term "halo(C₁-C₁₂)alkyl group" means a substituted and linear or branched alkyl group of 1 to 12 carbon atoms having as the substituent(s) one or more halogen atoms which may be the same or different. The term "(C₂-C₆)alkenyl group" means a linear or branched alkenyl group of 2 to 6 carbon atoms having a double bond. The term "halo(C₂-C₆)alkenyl group" means a substituted and linear or branched alkenyl group of 2 to 6 carbon atoms having as the substituent(s) one or more halogen atoms which may be the same or different. The term "(C₂-C₆)alkynyl group" means a linear or branched alkynyl group of 2 to 6 carbon atoms having a triple bond. The term "halo(C₂-C₆)alkynyl group" means a substituted and linear or branched alkynyl group of 2 to 6 carbon atoms having as the substituent(s) one or more halogen atoms which may be the same or different.

The term "5- or 6-membered heterocyclic ring having one or more heteroatoms which may be the same or different and are selected from the group consisting of oxygen atom, sulfur atom and nitrogen atom" means any heterocyclic ring derived from furan, thiophene, pyrrole, oxazole, thiazole, isothiazole, pyrazole, imidazole, 1,2,3-thiadiazole, 1,2,4-thiadiazole, 1,2,5-thiadiazole, 1,3,4-thiadiazole, 1,2,4-triazole, pyridine, pyridazine, pyrimidine, pyrazine, pynolidine, piperidine, morpholine, thiamorpholine, dithiolane, dithian, piperazine, dioxelan, imidazolizine and the like.

Preferable examples of the substituents of the 1,2,3-thiadiazole derivative of the general formula (I) used in the present invention are as follows. There is preferably used a compound in which R¹ is a (C₁-C₁₂)-alkyl group, halo(C₁-C₁₂)alkyl group, (C₃-C₆)cycloalkyl group, (C₂-C₁₂)alkenyl group, halo(C₂-C₁₂)alkenyl group, (C₂-C₁₂)alkynyl group, halo(C₂-C₁₂)alkynyl group, unsubstituted phenyl group or substituted phenyl group, and R² is -X-R¹². There is more preferably used a compound in which R¹ is a (C₁-C₆)alkyl group such as methyl, ethyl, n-propyl, i-propyl, n-butyl, i-butyl, s-butyl, t-butyl, n-pentyl or n-hexyl, and R² is a hydroxyl group, its salt, a thiol group, its salt, an amide group, or an anilide group.

There is most preferably used a compound in which R¹ is a methyl group and R² is a hydroxyl group, its salt, a thiol group, its salt, an amide group, or an anilide group.

As the salt of the 1,2,3-thiadiazole derivative of the general formula (I), there can be exemplified salts with alkali metals such as sodium, or potassium; salts with alkaline earth metals such as calcium or magnesium; unsubstituted ammonium salt; substituted ammonium salts having one or more substituents which may be the same or different and are selected from the group consisting of (C₁-C₁₂)alkyl groups, unsubstituted phenyl group, substituted phenyl groups, unsubstituted benzyl group and substituted benzyl groups; and guanidium salt.

The 1,2,3-thiadiazole derivative of the general formula (I) or salt thereof used as the active ingredient of the agricultural and horticultural disease controller of the present invention can be produced, for example, by any of the processes exemplified below: 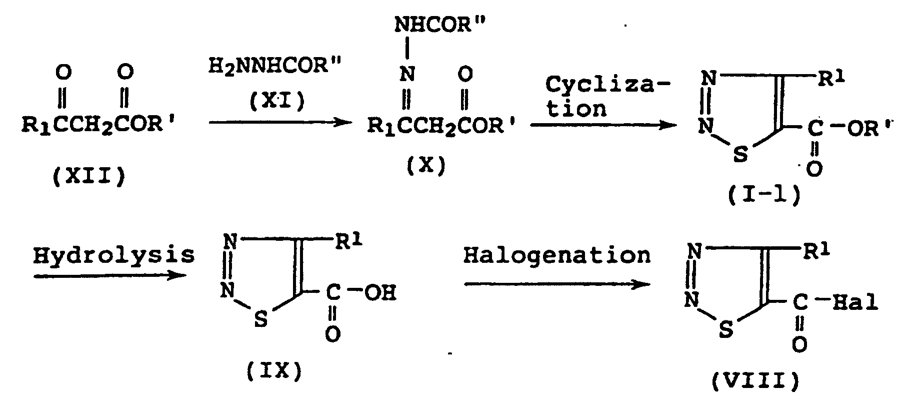 wherein R¹ is as defined above, R' is a (C₁-C₆)alkyl group, R" is an amino group or a (C₁-C₆)alkyl group, and Hal is a halogen atom.

A compound of the general formula (XII) is reacted with a compound of the general formula (XI) to obtain a compound of the general formula (X). The compound (X) is cyclized after or without isolation to obtain a 1,2,3-thiadiazole derivative of the general formula (I-1). The derivative (I-1) is hydrolyzed after or without isolation to obtain a compound of the general formula (IX). The compound (IX) is halogenated after or without isolation, whereby the acid halide of the general formula (VIII) can be produced.

The compound of the general formula (XII) can be produced by the process described in J. Org. Chem., 43, 2087 (1978), Formation of C-C bonds, Vol. 3, p. 259, 1979 (Georg Thime Publishers, Stuttgart), etc.

From the acid halide of the general formula (VIII) produced by the above process, the 1,2,3-thiadiazole derivative of the general formula (I) or salt thereof used as the active ingredient of the agricultural and horticultural disease controller of the present invention can be produced, for example, by any of the processes illustrated below.

### Production process 1.

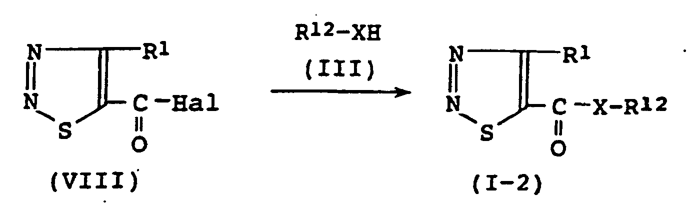

wherein R¹, R¹² and Hal are as defined above.

The acid halide of the general formula (VIII) is reacted with an alcohol of the general formula (III) to obtain a 1,2,3-thiadiazole derivative of the general formula (I-2).

Typical compounds as the 1,2,3-thiadiazole derivative of the general formula (I) or a salt thereof are listed in Table 1 but they are not intended in any way to limit the scope of the present invention.

The abbreviations in the R¹ column and R² column in Table 1 stand for the following compounds:

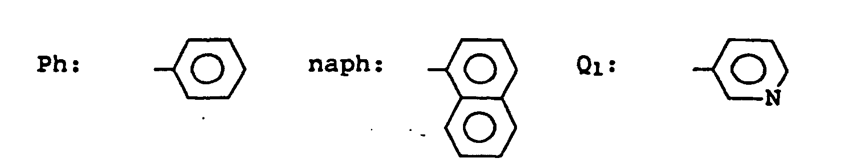

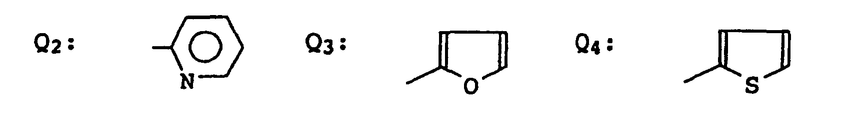

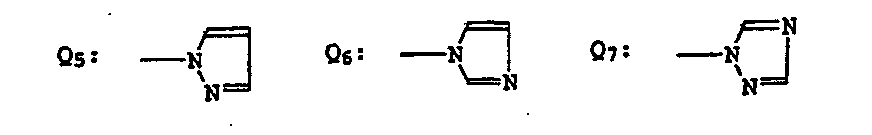

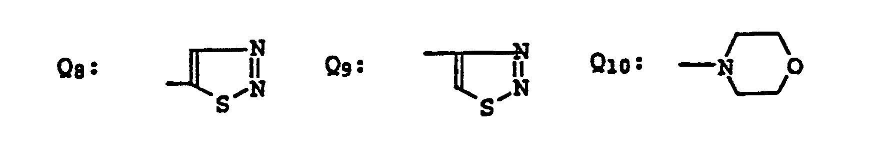

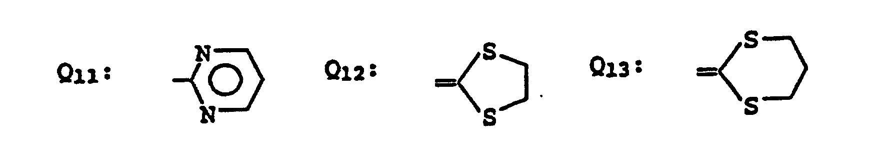

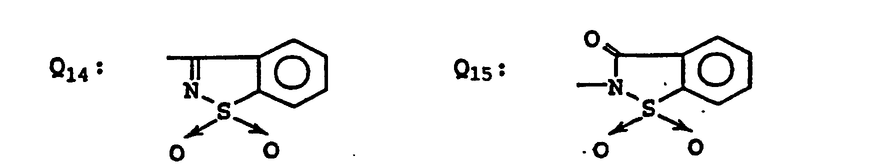

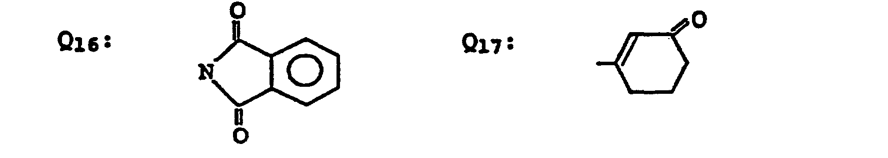

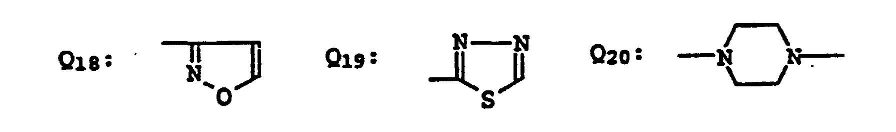

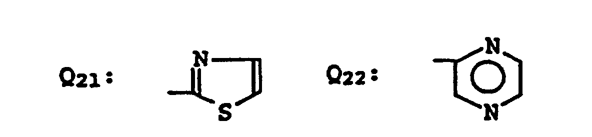

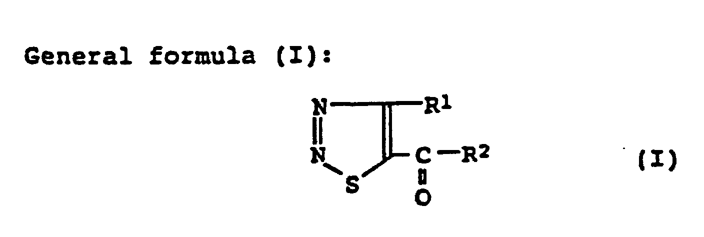

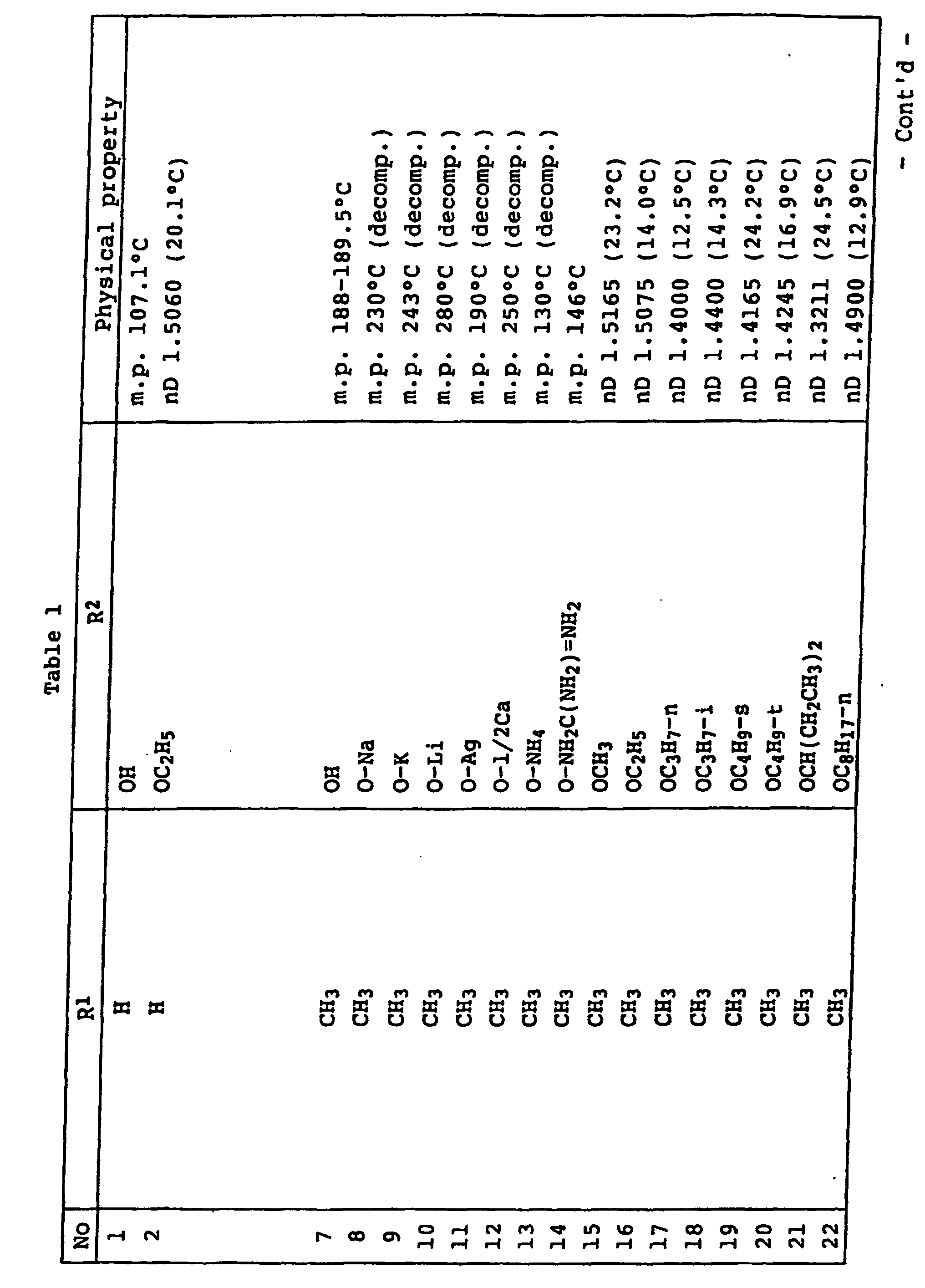

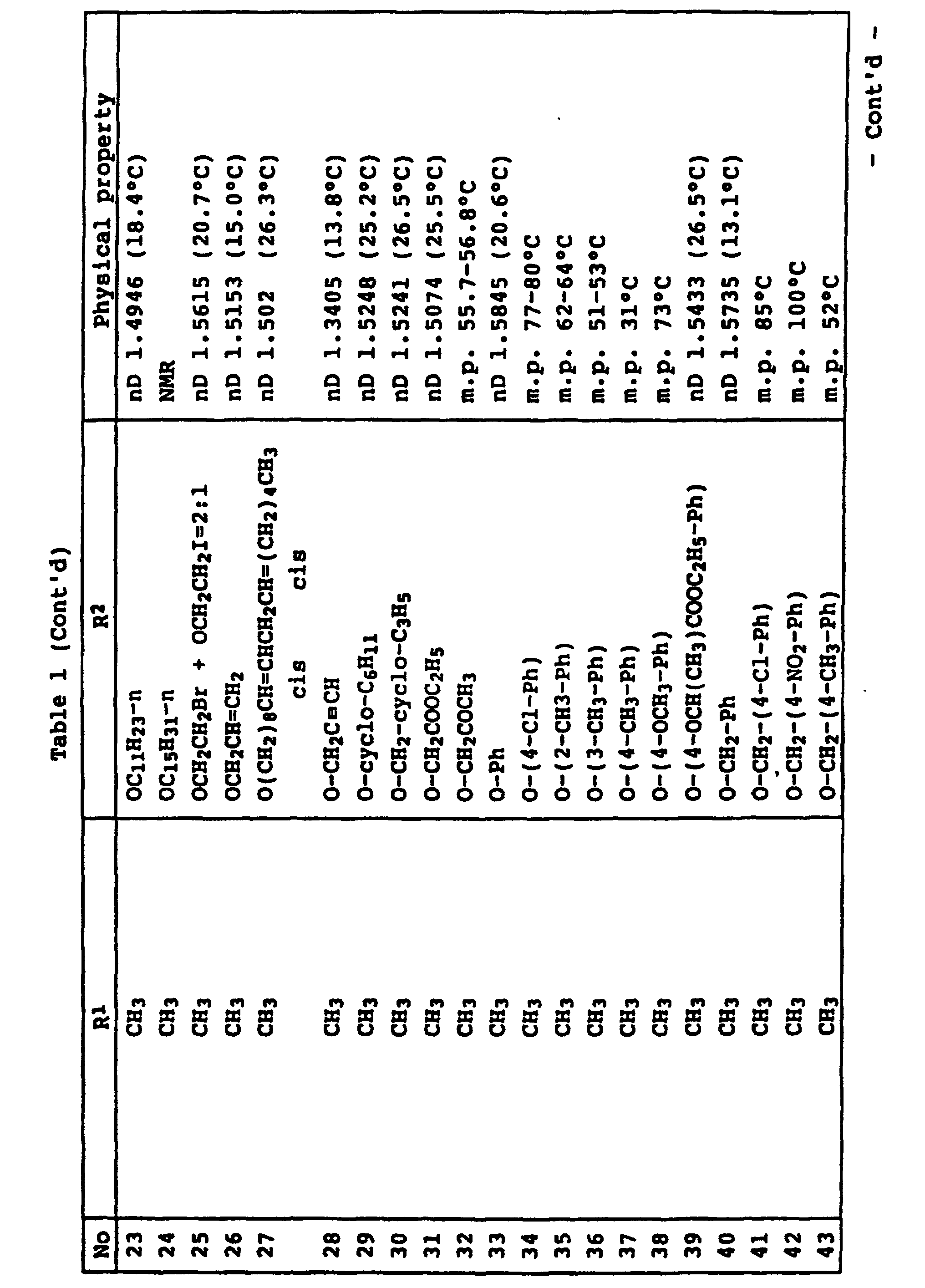

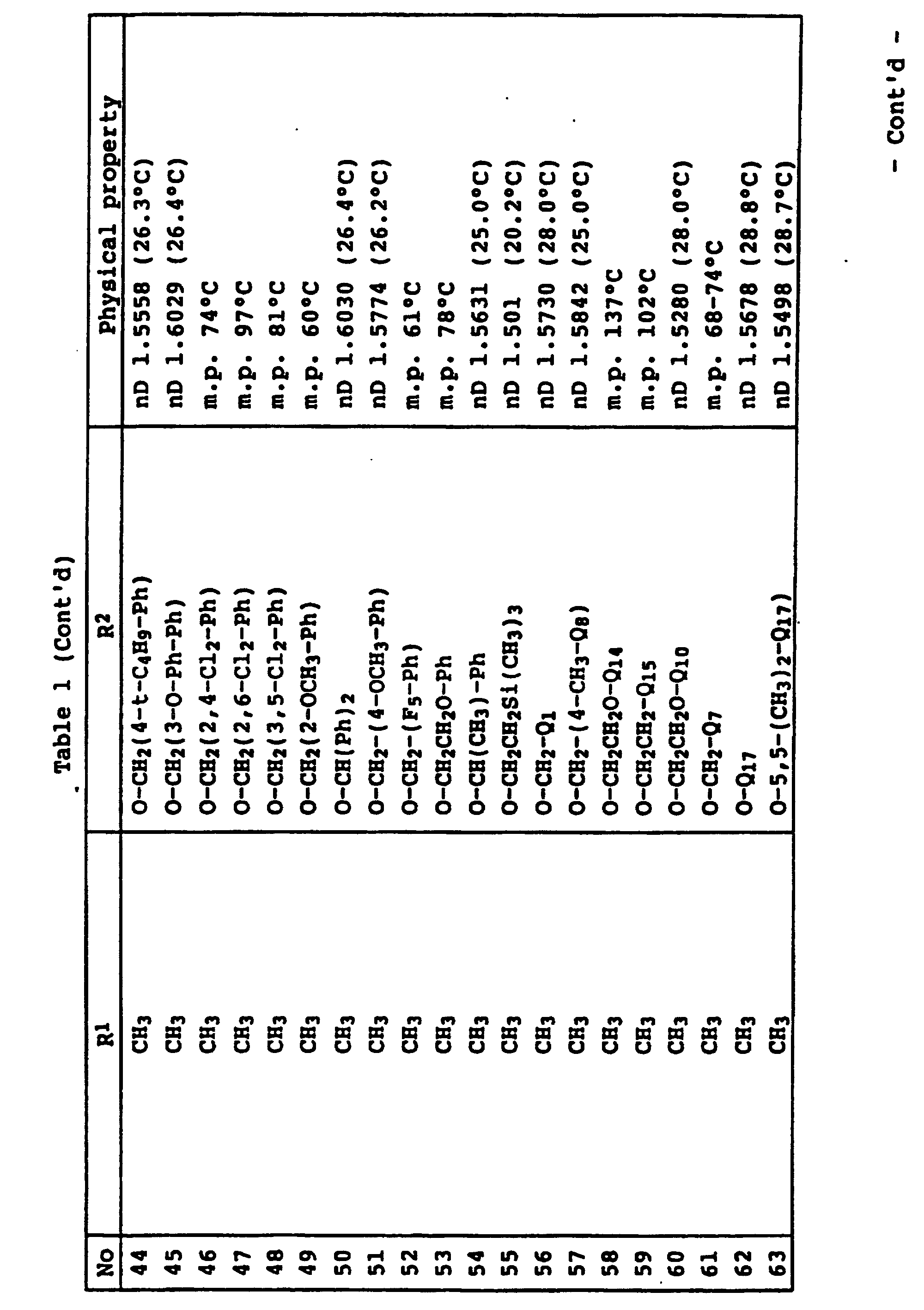

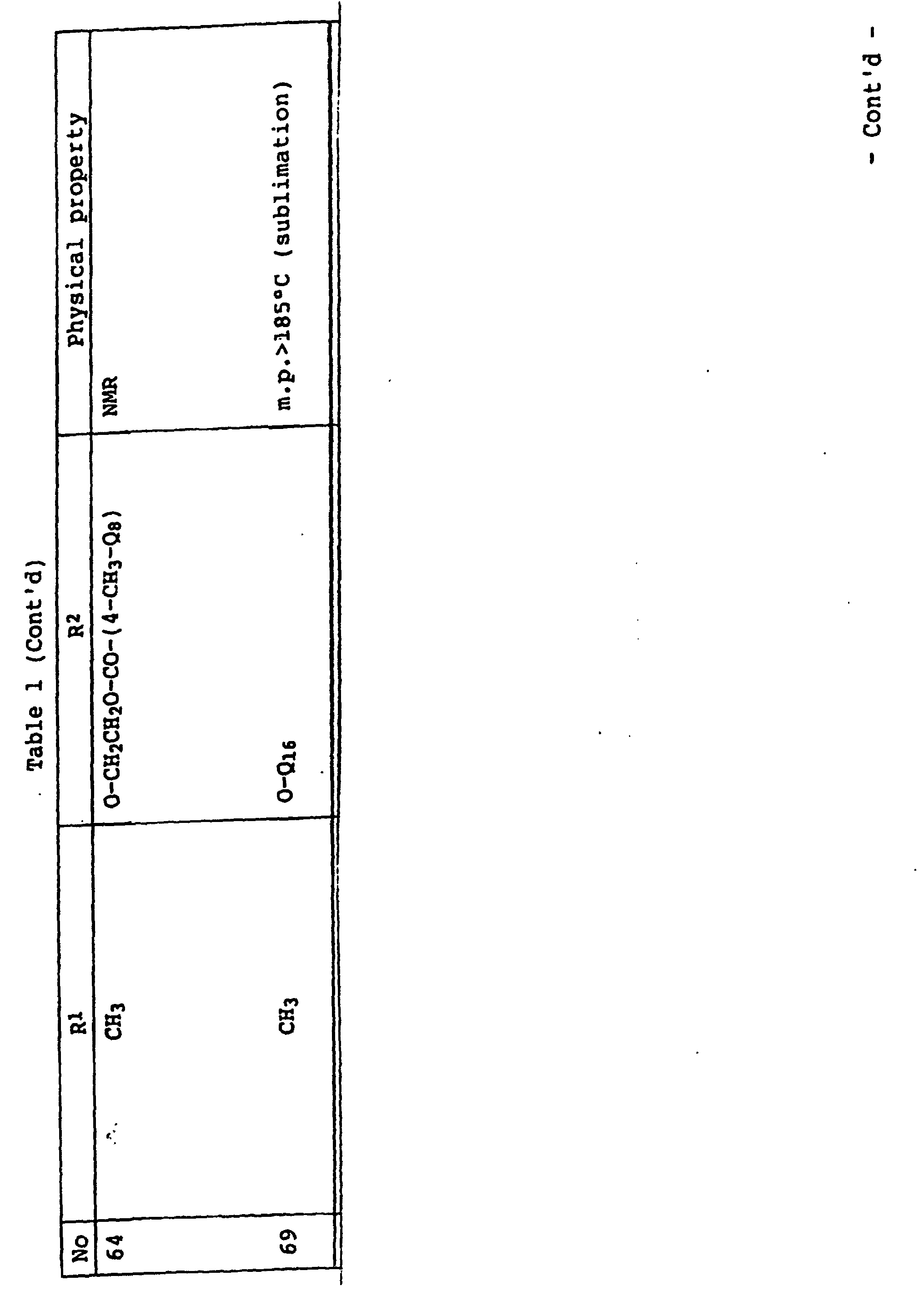

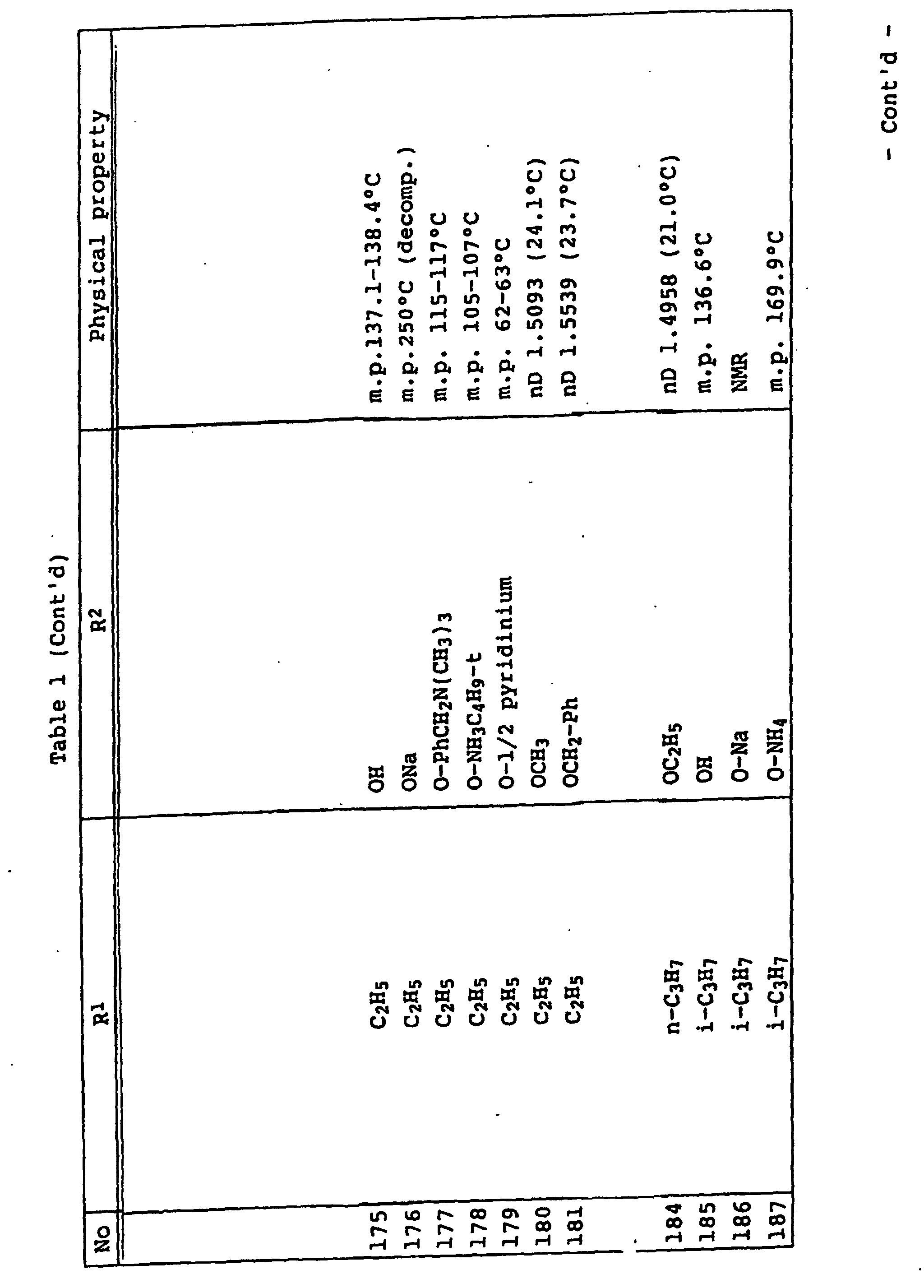

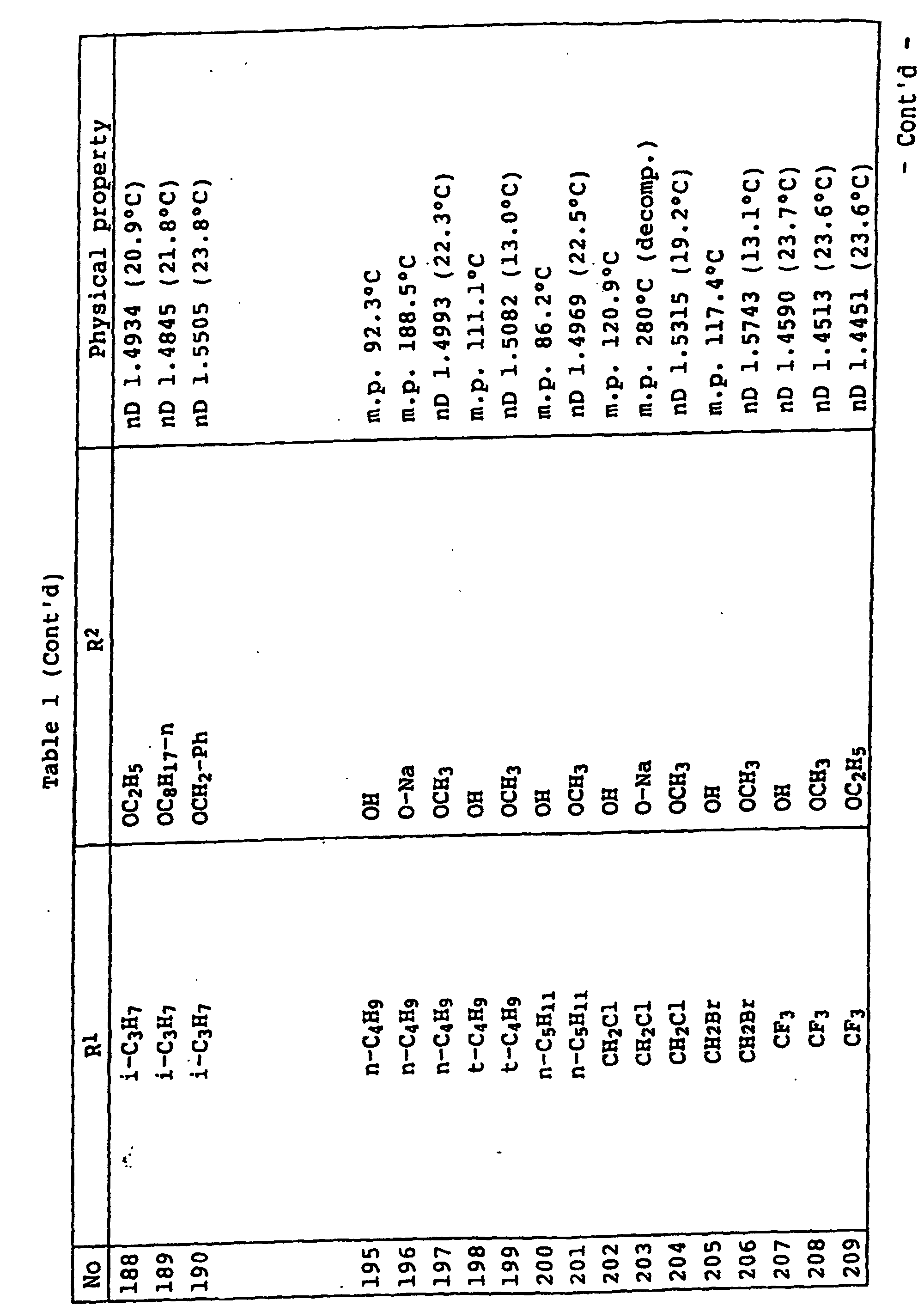

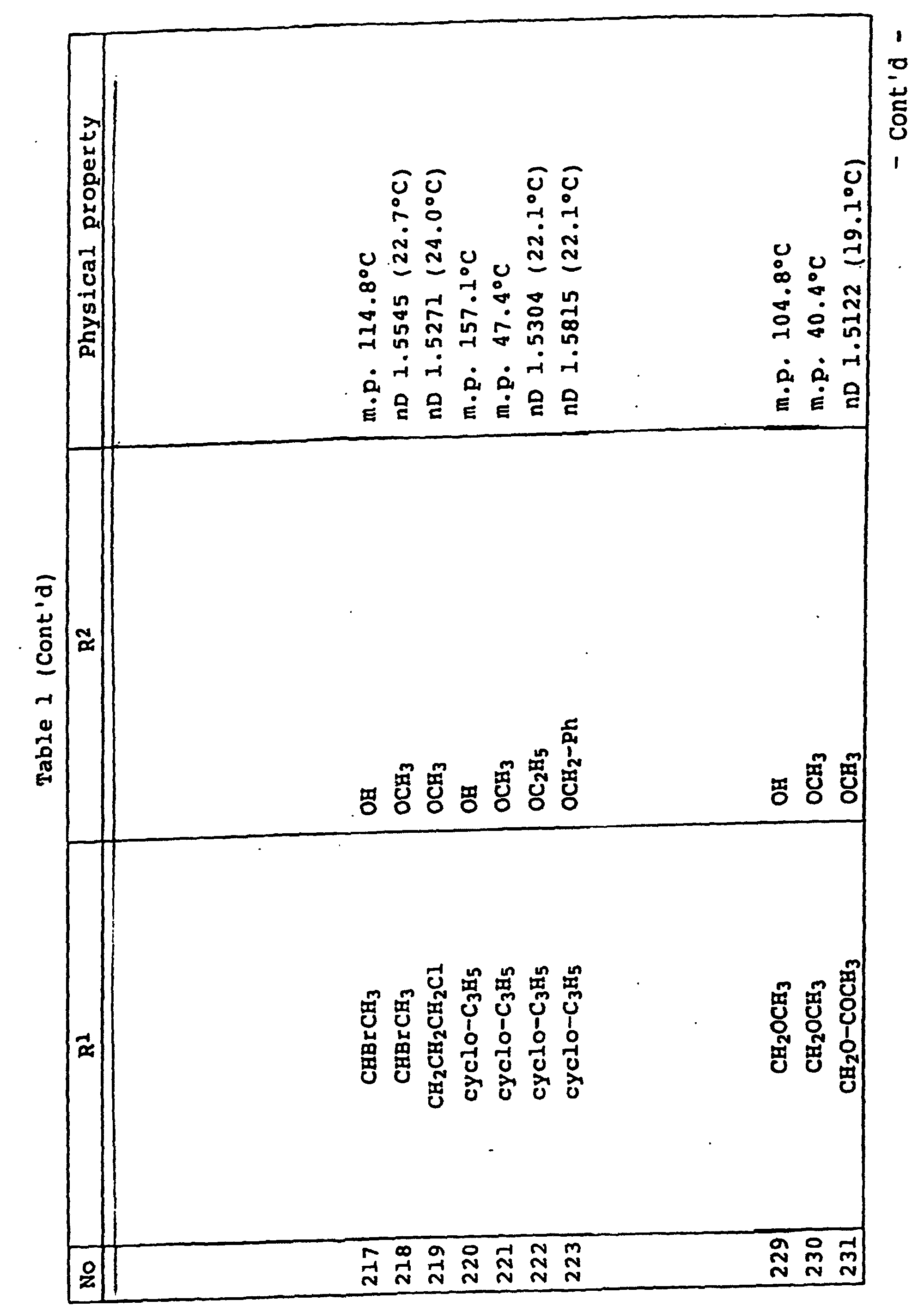

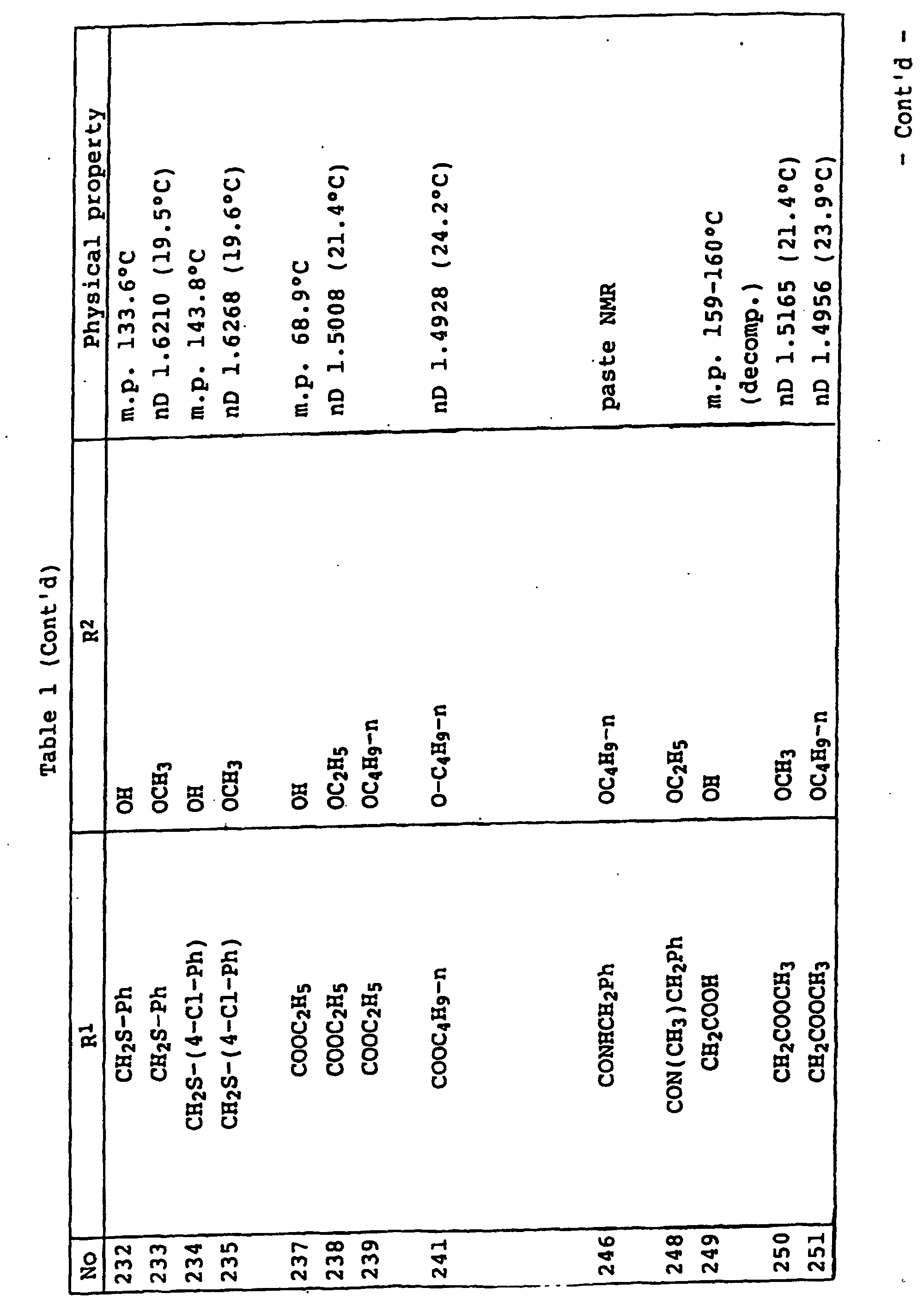

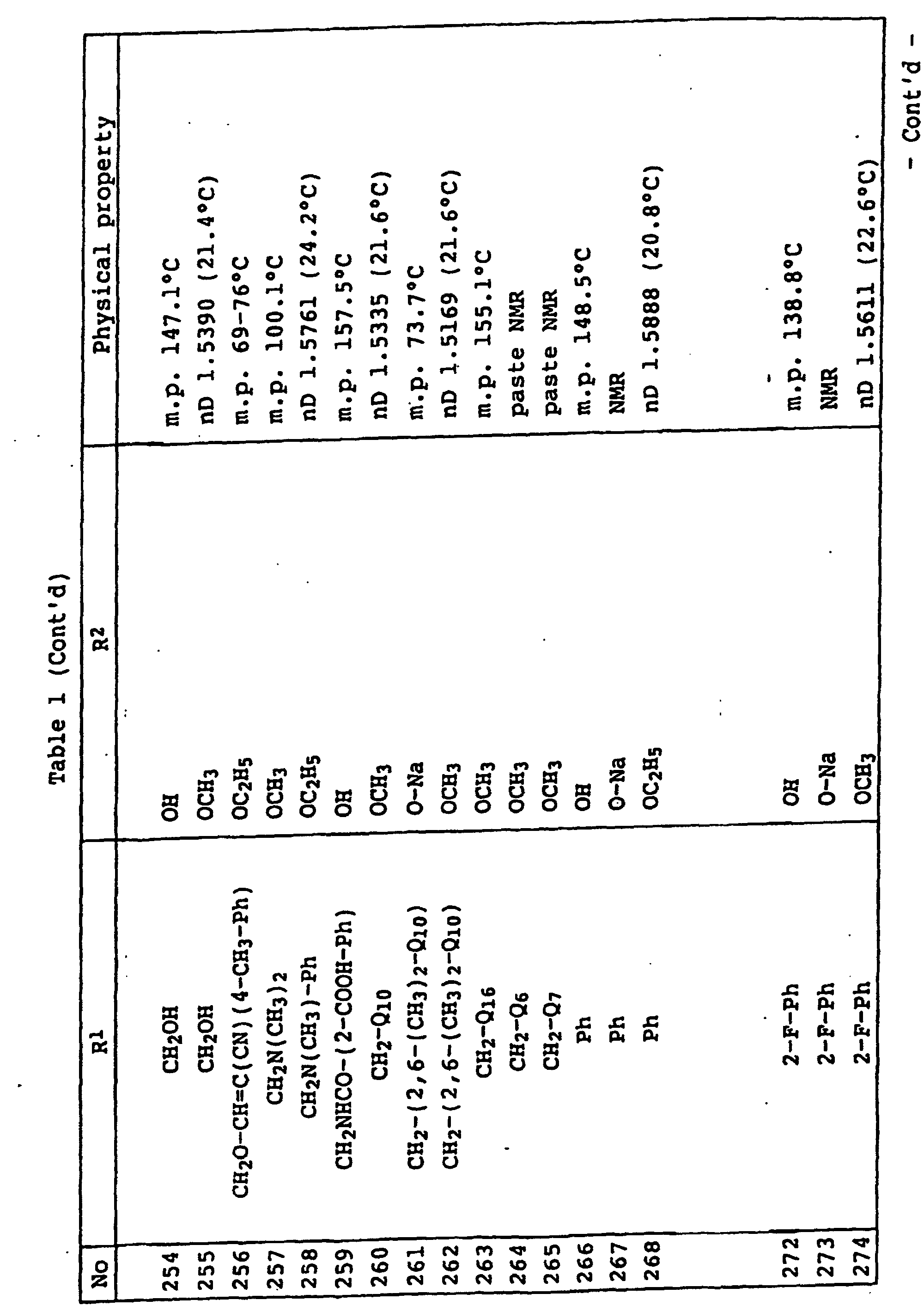

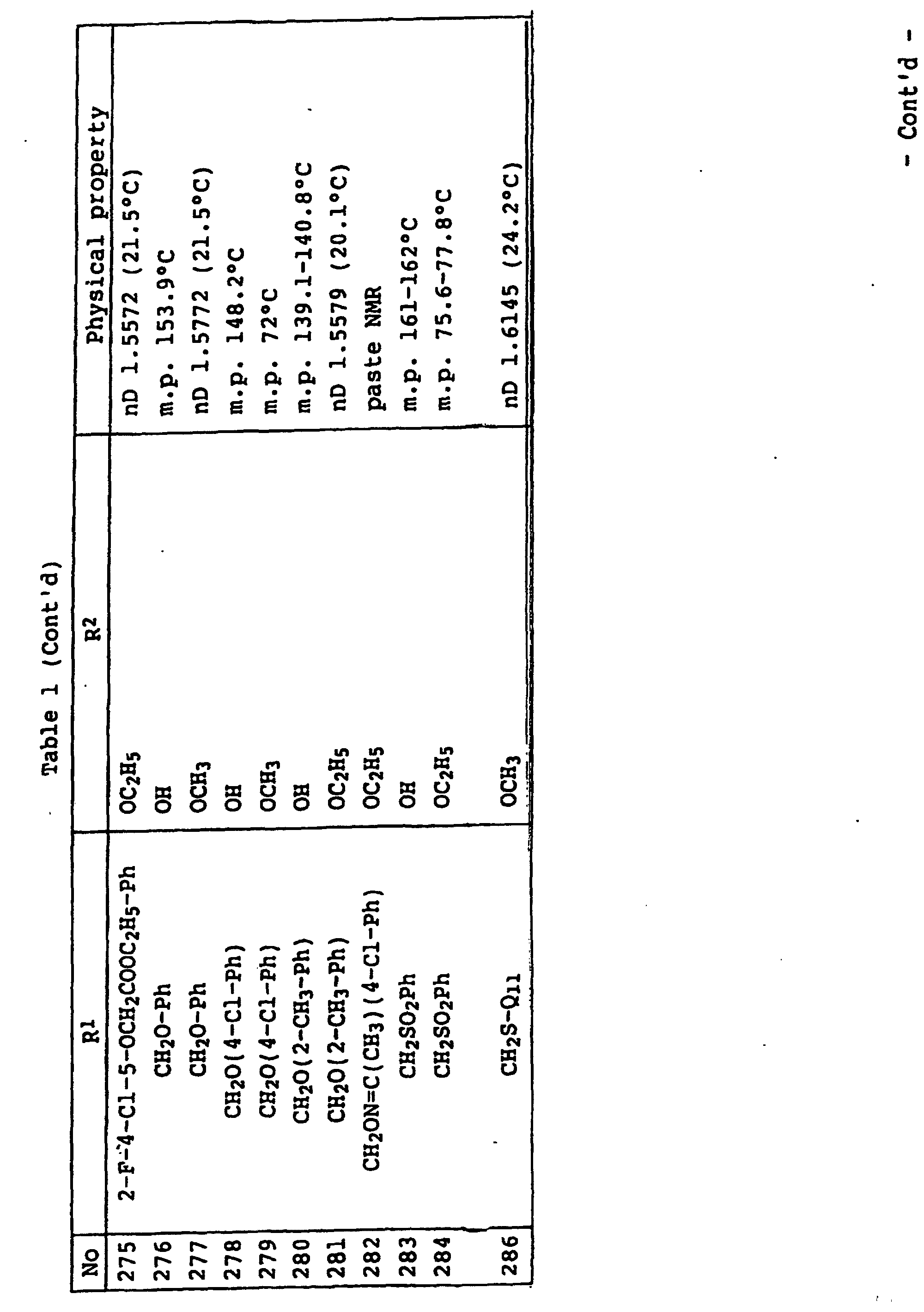

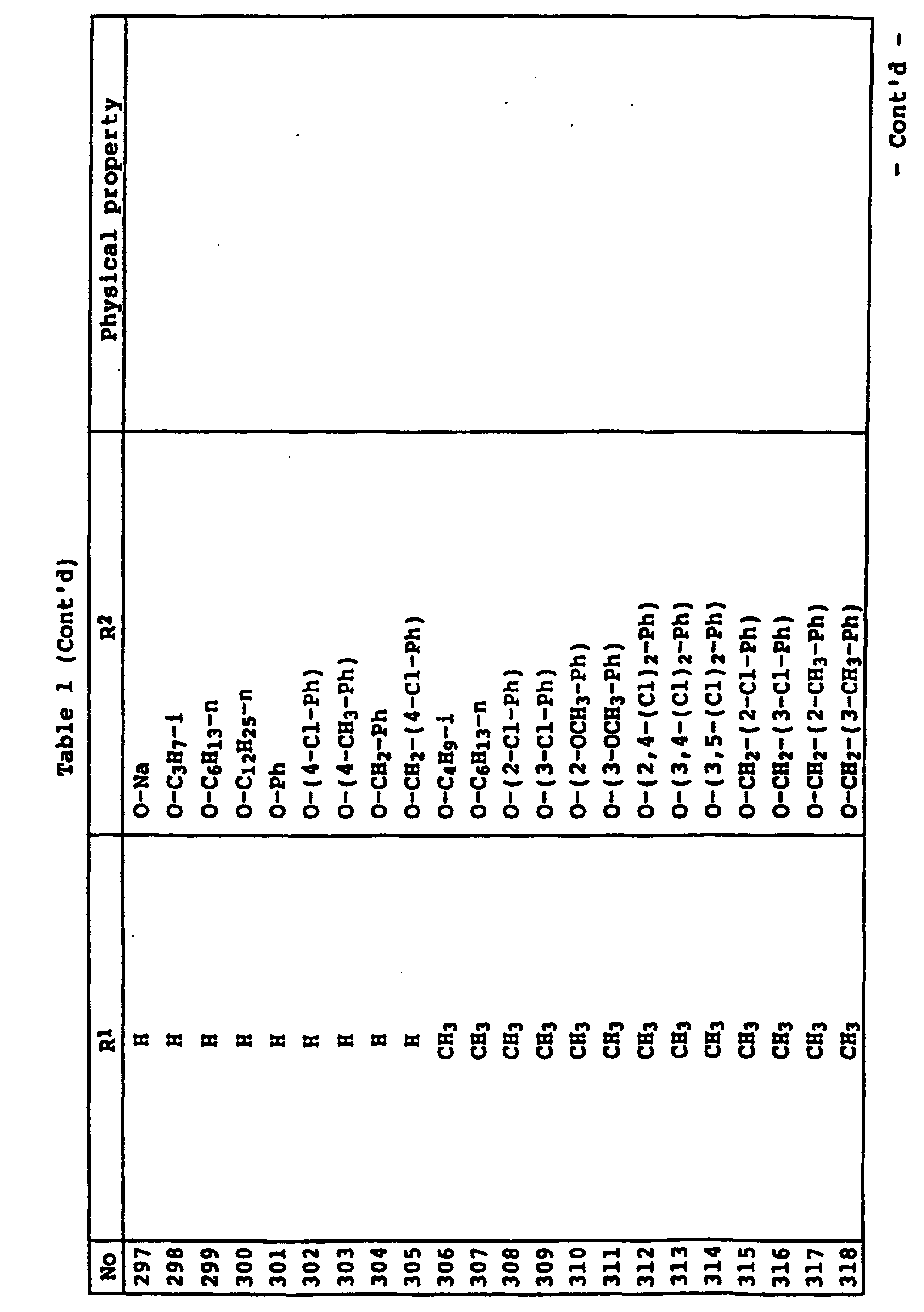

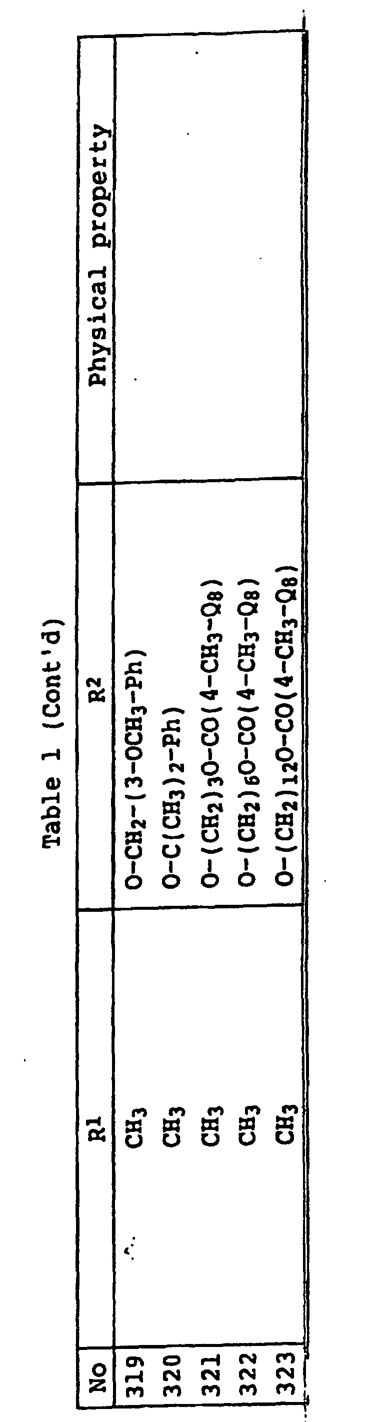

Table 2 shows ¹H-NMR data of the 1,2,3-thiadiazole derivatives having a physical property expressed by the word "oil", "paste" or "NMR" in Table 1.

**Table 2**

| No | ¹H-NMR [CDCl₃/TMS, δ value (ppm) |
|---|---|
| 24 | 0.879 (5, 3H), 1.255 (br, 8H), 1.75 (m, 2H), 2.974 (s, 1H), 4.340 (t, 2H). |
| 64 | 2.97 (s, 6H), 4.68 (s, 4H). |
| 186 | 1.33 (d, 6H), 4.13 (m, 1H). |
| 246 | 0.98 (t, 3H), 1.49 (m, 2H), 1.84 (m, 2H), 4.50 (t, 2H), 4.63 (d, 2H), 7.3-7.6 (m, 5H), 10.23 (br, s, 1H). |
| 264 | 4.000 (s, 3H), 5.854 (s, 2H), 7.039 (s, 1H), 7.142 (s, 1H), 7.742 (s, 1H). |
| 265 | 4.001 (s, 3H), 6.104 (s, 2H), 7.934 (s, 1H), 8.378 (s, 1H). |
| 267 | 7.3-7.5 (m, 3H), 8.12 (g, 2H) |
| 273 | 7.1-7.5 (m, 4H) |
| 282 | 1.41 (t, 3H), 2.27 (s, 3H), 4.44 (q, 2H), 5.88 (s, 2H), 7.2-7.6 (m, 4H). |

The 1,2,3-thiadiazole derivatives of the general formula (I) or salts thereof according to the present invention are useful for agricultural and horticultural disease control. For example, the compounds listed in Table 1 are very effective in controlling various diseases, for instance, rice blast (Pyricularia oryzae), rice sheath blight (Rhizoctonia solani), rice helminthosporium leaf spot (Cochliobolus miyabeanus), powdery mildew of various host plants, such as powdery mildew of barley and wheat (Erysiphe graminis), oats crown rust (Puccinia coronata), rust of other plants, tomato late blight (Phytophthora infestans), late blight or Phytophthora rots of other plants, downy mildew of various plants, such as cucumber downy mildew (Pseudoperonospora cubensis) and grape downy mildew (Plasmopara viticola), apple scab (Venturia inaequalis), apple alternaria leaf spot (Alternaria mali), pear black spot (Alternaria kikuchiana), citrus melanose (Diaporthe citri), cucumber bacterial blight (Pseudomonas syringae pv. lachrymans), tomato bacterial wilt (Pseudomonas solanacearum), cabbage black rot (Xanthomonas campestris), citrus canker (Xanthomonas citri (Hasse) Dowson), rice bacterial leaf blight (Xanthomonas oryzae), cabbage bacterial soft rot (Erwinia carotovora), and tobacco mosaic (Tobacco mosaic virus).

The agricultural and horticultural disease controller of the present invention is markedly effective in controlling the above-exemplified diseases which damage paddy field crops, upland crops, fruit trees, vegetables, other crops, flowers and ornamental plants, and the like. Therefore, the desired effects of the agricultural and horticultural disease controller of the present invention can be obtained by applying the disease controller to the paddy field water, stalks and leaves of fruit trees, vegetables, other crops, flowers and ornamental plants, soil, etc., at a season at which the diseases are expected to occur, before their occurrence or at the time when their occurrence is confirmed.

In general, the agricultural and horticultural disease controller of the present invention is used after being prepared into a conveniently usable form according to an ordinary manner for preparation of agrochemicals.

That is, the 1,2,3-thiadiazole derivative of the general formula (I) or salt thereof according to the present invention and, optionally, an adjuvant are blended with a suitable inert carrier in a proper proportion and prepared into a suitable preparation form such as a suspension, emulsifiable concentrate, soluble concentrate, wettable powder, granules, dust or tablets through dissolution, dispersion, suspension, mixing, impregnation, adsorption or sticking.

The inert carrier used in the present invention may be either solid or liquid. As the solid carrier, there can be exemplified soybean flour, cereal flour, wood flour, bark flour, saw dust, powdered tobacco stalks, powdered walnut shells, bran, powdered cellulose, extraction residue of vegetables, powdered synthetic polymers or resins, clays (e.g. kaolin, bentonite, and acid clay), talcs (e.g. talc and pyrophyllite), silica powders or flakes (e.g. diatomaceous earth, silica sand, mica and white carbon, i.e. synthetic, high-dispersion silicic acid, also called finely divided hydrated silica or hydrated silicic acid, some of commercially available products contain calcium silicate as the major component), activated carbon, powdered sulfur, powdered pumice, calcined diatomaceous earth, ground brick, fly ash, sand, calcium carbonate powder, calcium phosphate powder and other inorganic or mineral powders, chemical fertilizers (e.g. ammonium sulfate, ammonium phosphate, ammonium nitrate, urea and ammonium chloride), and compost. These carriers may be used alone or as a mixture thereof.

The liquid carrier is that which itself has solubility or which is without such solubility but is capable of dispersing an active ingredient with the aid of an adjuvant. The following are typical examples of the liquid carrier and can be used alone or as a mixture thereof. Water; alcohols such as methanol, ethanol, isopropanol, butanol and ethylene glycol; ketones such as acetone, methyl ethyl ketone, methyl isobutyl ketone, diisobutyl ketone and cyclohexanone; ethers such as ethyl ether, dioxane, Cellosolve, dipropyl ether and tetrahydrofuran; aliphatic hydrocarbons such as kerosene and mineral oils; aromatic hydrocarbons such as benzene, toluene, xylene, solvent naphtha and alkylnaphthalenes; halogenated hydrocarbons such as dichloroethane, chloroform, carbon tetrachloride and chlorobenzene; esters such as ethyl acetate, diisopropyl phthalate, dibutyl phthalate and dioctyl phthalate; amides such as dimethylformamide, diethylformamide and dimethylacetamide; nitriles such as acetonitrile; and dimethyl sulfoxide.

The following are typical examples of the adjuvant, which are used depending upon purposes and used alone or in combination in some cases, or need not to be used at all.

To emulsify, disperse, dissolve and/or wet an active ingredient, a surfactant is used. As the surfactant, there can be exemplified polyoxyethylene alkyl ethers, polyoxyethylene alkylaryl ethers, polyoxyethylene higher fatty acid esters, polyoxyethylene resinates, polyoxyethylene sorbitan monolaurate, polyoxyethylene sorbitan monooleate, alkylarylsulfonates, naphthalenesulfonic acid condensation products, ligninsulfonates and higher alcohol sulfate esters.

Further, to stabilize the dispersion of an active ingredient, tackify it and/or bind it, there may be used adjuvants such as casein, gelatin, starch, methyl cellulose, carboxymethyl cellulose, gum arabic, polyvinyl alcohols, turpentine, bran oil, bentonite and ligninsulfonates.

To improve the flowability of a solid product, there may be used adjuvants such as waxes, stearates and alkyl phosphates.

Adjuvants such as naphthalenesulfonic acid condensation products and polycondensates of phosphates may be used as a peptizer for dispersible products.

Adjuvants such as silicon oils may also be used as a defoaming agent.

The content of the active ingredient may be varied as required. In dusts or granules, the suitable content thereof is from 0.01 to 50% by weight. In emulsifiable concentrates or flowable wettable powders, it is also from 0.01 to 50% by weight.

The present inventive agricultural and horticultural disease controller containing the 1,2,3-thiadiazole derivative of the general formula (I) or a salt thereof as an active ingredient is used to control various diseases in the following manner. That is, it is applied to a crop on which the diseases are expected to occur, or a site where the occurrence of the diseases is undesirable, as it is or after being properly diluted with or suspended in water or the like, in an amount effective for control of the diseases. For example, to control the diseases of paddy rice, said disease controller can be used by a method such as submerged application to a regular paddy field, application to a rice nursery bed, dressing of seeds for direct sowing on flooded paddy field, or seed disinfection.

The applying dosage of the present inventive agricultural and horticultural disease controller containing the 1,2,3-thiadiazole derivative of the general formula (I) or a salt thereof as an active ingredient is varied depending upon various factors such as a purpose, diseases to be controlled, a growth state of a plant, tendency of disease occurrence, weather, environmental conditions, a preparation form, an application method, an application site and application time. It may be properly chosen in the range of 0.1 g to 10 kg (in terms of the active ingredient) per 10 ares depending upon purposes.

The present inventive agricultural and horticultural disease controller containing the 1,2,3-thiadiazole derivative of the general formula (I) or a salt thereof as an active ingredient may be used in admixture with other agricultural and horticultural disease controllers in order to expand both spectrum of controllable diseases and the period of time when effective applications are possible or to reduce the dosage.

Typical examples and test examples of the present invention are described below.

In the examples, parts are all by weight.

### Example 1

| | |
|---|---|
| Each compound listed in Table 1 | 50 parts |
| Xylene | 40 parts |
| Mixture of polyoxyethylene nonylphenyl ether and calcium alkylbenzenesulfonate | 10 parts |

An emulsifiable concentrate was prepared by mixing uniformly the above ingredients to effect dissolution.

### Example 2

| | |
|---|---|
| Each compound listed in Table 1 | 3 parts |
| Clay powder | 82 parts |
| Diatomaceous earth powder | 15 parts |

A dust was prepared by mixing uniformly and grinding the above ingredients.

### Example 3

| | |
|---|---|
| Each compound listed in Table 1 | 5 parts |
| Mixed powder of bentonite and clay | 90 parts |
| Calcium lignin sulfonate | 5 parts |

Granules were prepared by mixing the above ingredients uniformly, and kneading the resulting mixture together with a suitable amount of water, followed by granulation and drying.

### Example 4

| | |
|---|---|
| Each compound listed in Table 1 | 20 parts |
| Mixture of kaolin and synthetic, high-dispersion silicic acid | 75 parts |
| Mixture of polyoxyethylene nonylphenyl ether and calcium alkylbenzenesulfonate | 5 parts |

A wettable powder was prepared by mixing uniformly and grinding the above ingredients.

### Test Example 1

### Controlling effect on rice blast by submerged application

Rice plants at the 5 to 6 leaf stage cultivated in a 1/10000-are pot were subjected to submerged application of a chemical containing each compound listed in Table 1 as an active ingredient, in a dosage of 200 g/10 a in terms of the active ingredient. After standing in a greenhouse for 1 week, the plants were inoculated with a suspension of spores of blast fungus (Pyricularia oryzae) by spraying.

After the inoculation, the plants were allowed to stand in a moist chamber for 1 day and then a greenhouse for 6 days to cause the disease sufficiently. Then, lesions in each leaf were counted and then compared with those on the untreated plot, and the controlling degree was calculated, whereby the effect was judged according to the following criterion.

| Effect | Controlling degree (%) |
|---|---|
| A | 100 - 95 |
| B | 94 - 85 |
| C | 84 - 60 |
| D | 59 - 0 |

As a result of the above test, the compounds listed in Table 1 were found to have a marked blast-controlling activity. Of these compounds, the following were rated C or higher: compound Nos. 1 to 42, 44 to 61, 62 to 69, 175 to 190, 195 to 196, 198 to 200, 202 to 222, 229 to 233, 237, 254 to 255, 268, 272, 273, 276 and 278. In particular, the following were rated A, namely, the following had an excellent blast-controlling activity: compound Nos. 1, 7 to 19, 21, 24 to 26, 28, 29, 32, 33, 37 to 40, 52, 54, 55, 57 to 59, 64, 175 to 190, 198, 199, 202, 208 to 209, 217 to 221, 229, 233, 237, and 278.

### Test Example 2

### Controlling effect on barley powdery mildew

Barley plants at the 3.5 leaf stage cultivated in a pot were applied with a spray mix containing each compound listed in Table 1 as an active ingredient at the concentration of 200 ppm. After standing thus treated plants in a greenhouse for 1 week, the plants were inoculated with spores of powdery mildew fungus (Erysiphe graminis f. sp. hordei)

After the inoculation, the plants were allowed to stand in a greenhouse for 1 week to cause the disease sufficiently. Then, lesions in each leaf were counted and then compared with those on the untreated plot, and the controlling degree was calculated, whereby the effect was judged according to the following criterion.

| Effect | Controlling degree (%) |
|---|---|
| A | 100 - 80 |
| B | 79 - 60 |
| C | 59 - 0 |

As a result of the above test, the compounds listed in Table 1 were found to have a marked powdery mildew-controlling activity. Of these compounds, the following were rated B or higher: compound Nos. 7 to 9, 15, 16, 33, 40, 180, 217 and 218. In particular, the following were rated A: compound Nos. 7 to 9, 15, 16, 33, 40, 180, 217 and 218.

### Referential Production Example 1

### Production of sodium 4-ethyl-1,2,3-thiadiazole-5-carboxylate (compound No. 8)

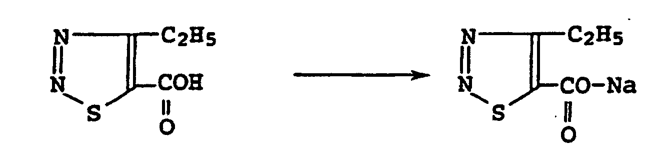

To a solution of 0.13 g of sodium hydroxide in 3 ml of ethanol was added 0.5 g of 4-ethyl-1,2,3-thiadiazole-5-carboxylic acid, and the reaction was carried out at room temperature for 24 hours.

After completion of the reaction, the solvent was distilled off under reduced pressure and the crude product thus obtained was recrystallized from ethanol to obtain 0.44 g of the desired compound.
Physical property: m.p. 250°C (decomp.).
Yield: 77%.

### Referential Production Example 2

### Production of t-butylammonium 4-ethyl-1,2,3-thiadiazole-5-carboxylate (compound No. 185)

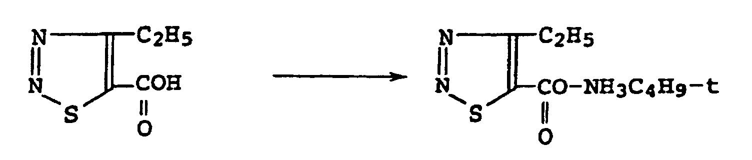

In 3 ml of ethanol was dissolved 0.4 g of 4-ethyl-1,2,3-thiadiazole-5-carboxylic acid, after which 0.19 g of t-butylamine was added to the solution and the reaction was carried out at room temperature for 24 hours.

After completion of the reaction, the crystals precipitated in the reaction solution were collected by filtration and washed with n-hexane to obtain 0.54 g of the desired compound.
Physical property: m.p. 105 - 107°C.
Yield: 93%.

### Referential Production Example 3

### Production of ethyl 4-(2-methylphenoxymethyl)-1,2,3-thiadiazole-5-carboxylate (compound No. 281)

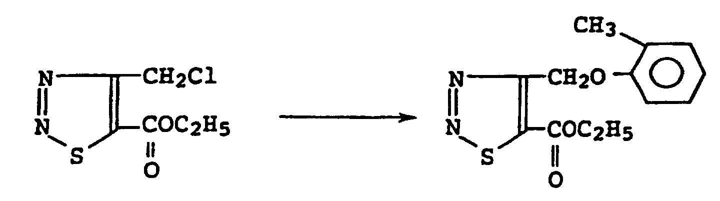

In 20 ml of dimethylformamide was suspended 0.5 g of sodium hydride, after which 1.57 g of 2-methylphenol was added to the suspension and the resulting mixture was stirred at room temperature for 5 minutes. The reaction mixture was cooled with ice, followed by adding thereto 3 g of ethyl 4-chloromethyl-1,2,3-thiadiazolecarboxylate, and the resulting mixture was stirred at room temperature and allowed to stand for 24 hours.

After completion of the reaction, the reaction mixture was poured into ice water and the desired compound was extracted three times with ethyl acetate. The extracted solution was washed with water, dried over anhydrous sodium sulfate, and then distilled under reduced pressure to remove the solvent, whereby 1.33 g of the desired compound was obtained.
Physical property: nD 1.5579 (20.1°C).
Yield: 33%.

### Referential Production Example 4

### Production of isopropyl 4-methyl-1,2,3-thiadiazole-5-carboxylate (compound No. 18)

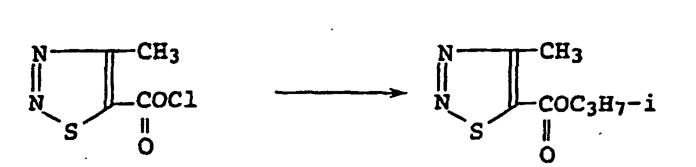

To 1.0 g (6.9 mmoles) of 4-methyl-1,2,3-thiadiazole-5-carboxylic acid were added 40 ml of thionyl chloride and 2 drops of dimethylformamide at room temperature, and the resulting mixture was refluxed with heating for 3 hours. After completion of the reaction, the reaction mixture was cooled to room temperature, and the excess thionyl chloride was distilled off and 7.0 ml of tetrahydrofuran was added to the residue. Then, 2.1 g (21 mmoles) of triethylamine and 0.83 g (13.8 mmoles) of isopropanol were added and the resulting mixture was stirred at room temperature for 18 hours, after which a saturated aqueous sodium chloride solution was added. The desired compound was extracted with ethyl acetate and the organic layer was washed successively with diluted hydrochloric acid, a saturated aqueous sodium hydrogencarbonate solution and a saturated aqueous sodium chloride solution, dried over anhydrous sodium sulfate, and then concentrated under reduced pressure. The resulting residue was purified by a silica gel column chromatography (n-hexane : ethyl acetate = 10 : 1) to obtain 1.0 g of the desired compound.
Physical property: nD 1.4400 (14.3°C).
Yield: 79%.

## Claims

1. Use of a composition which comprises an inert carrier and an active amount of a 1,2,3-thiadiazole derivative represented by the general formula (I), or a salt thereof for controlling agricultural and horticultural diseases which damage crops, fruit trees, vegetables, flowers and ornamental plants: 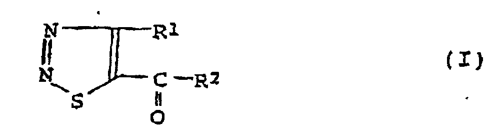 wherein R¹ is ① a hydragen atom; ② a (C₁-C₁₂)alkyl group, ③ a halo(C₁-C₁₂)alkyl group, ④ a (C₂-C₁₂)-alkenyl group, ⑤ a halo(C₂-C₁₂)alkenyl group, ⑥ a (C₂-C₁₂)alkynyl group, ⑦ a halo(C₂-C₁₂)alkynyl group, ⑧ a (C₃-C₆)cycloalkyl group, ⑨ an unsubstituted phenyl group, 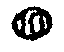 a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, 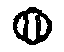 a group represented by the formula: 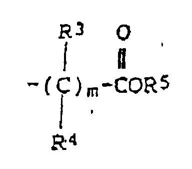 (wherein R³ and R⁴, which may be the same or different, are hydrogen atoms, (C₁-C₁₂)alkyl groups or halo(C₁-C₁₂)alkyl groups, m is zero or an integer of 1 to 6, and R⁵ is a hydrogen atom; a (C₁-C₁₂)alkyl group; a halo(C₁-C₁₂)alkyl group; a (C₂-C₁₂)alkenyl group; a (C₂-C₁₂)alkynyl group; an unsubstituted phenyl group; a substituted phenyl group having one or more substituents which may be the same or different and are selected from the group consisting of halogen atoms, nitro group, cyano group, (C₁-C₆)alkyl groups, halo(C₁-C₆)alkyl gxoups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted phenyl(C₁-C₆)alkyl group; or a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups), 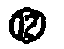 a group represented by the formula: 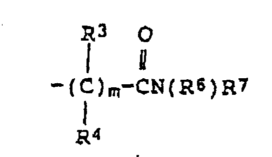 (wherein R³, R⁴ and m are as defined above, and R⁶ and R⁷, which may be the same or different, are hydrogen atoms; (C₁-C₁₂)alkyl groups; halo(C₁-C₁₂)alkyl groups; (C₂-C₁₂)alkenyl groups; (C₂-C₁₂)alkynyl groups; (C₁-C₁₂)alkoxy(C₁-C₆)alkyl groups; (C₁-C₁₂)alkylthio(C₁-C₆)alkyl groups; cyano(C₁-C₁₂)alkyl groups; unsubstituted phenyl groups; substituted phenyl groups having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; unsubstituted phenyl(C₁-C₆)alkyl groups; or substituted phenyl(C₁-C₆)alkyl groups having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; R⁶ and R⁷ being able to be taken together to represent a (C₄-C₆)alkylene group which may contain an oxygen atom between adjacent carbon atoms of the carbon chain), 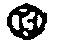 a group represented by the formula: 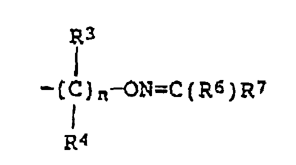 (wherein R³, R⁴, R⁶ and R⁷ are as defined above, and n is an integer of 1 to 6), or 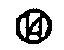 a group represented by the formula: 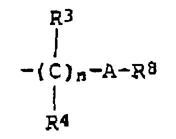 [wherein R³, R⁴ and n are as defined above, A is
-O- , 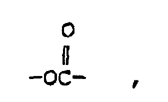 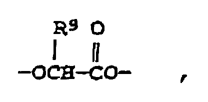
(wherein R⁹ is a hydrogen atom, a (C₁-C₁₂)alkyl group or a halo(C₁-C₁₂)alkyl group),
-S- ,
-SO- ,
-SO₂- , 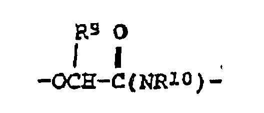
{wherein R⁹ is as defined above, and R¹⁰ is a hydrogen atom, a (C₁-C₁₂)alkyl group or a halo(C₁-C₁₂)alkyl group, R⁹ and R¹⁰ being able to be taken together to represent a (C₄-C₆)alkylene group which may contain, between adjacent carbon atoms of the carbon chain, an oxygen atom, a sulfur atom or
N-R¹¹
(wherein R¹¹ is a hydrogen atom, a (C₁-C₁₂)alkyl group or a halo(C₁-C₁₂)alkyl group)}, or
-N(R¹⁰)-
(wherein R¹⁰ is as defined above), and R⁸ is a hydrogen atom; a (C₁-C₁₂)alkyl group; a halo(C₁-C₁₂)alkyl group; a (C₂-C₁₂)alkenyl group; a (C₂-C₁₂)alkynyl group; a (C₁-C₁₂)alkoxy(C₁-C₁₂)alkyl group; a (C₁-C₁₂)alkylthio(C₁-C₁₂)alkyl group; a cyano(C₁-C₁₂)alkyl group; an unsubstituted phenyl group; a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted phenyl(C₁-C₆)-alkyl group; a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; an unsubstituted 5- or 6-membered heterocyclic ring having one or more heteroatoms which may be the same or different and are selected from the group consisting of oxygen atom, sulfur atom and nitrogen atom; or a substituted 5- or 6-membered heterocyclic ring having one or more heteroatoms which may be the same or different and are selected from the group consisting of oxygen atom, sulfur atom and nitrogen atom, said substituted heterocyclic ring having one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups], and
R² is a group represented by the formula:
-X-R¹²
[wherein X is an oxygen atom, and R¹²
is ① a hydrogen atom, ② a (C₁-C₁₂)alkyl group, ③ a halo(C₁-C₁₂)alkyl group, ④ a (C₂-C₂₀)alkenyl group, ⑤ a halo(C₂-C₂₀)alkenyl group, ⑥ a (C₂-C₁₂)alkynyl group, ⑦ a halo(C₂-C₁₂)alkynyl group, ⑧ a hydroxy(C₁-C₆)alkyl group, ⑨ a (C₁-C₁₂)alkoxy(C₁-C₁₂)alkyl group, 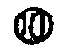 a (C₁-C₁₂)alkylthio(C₁-C₁₂)alkyl group, 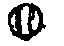 a (C₃-C₆)cycloalkyl group,
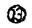 an unsubstituted phenyl group, 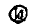 a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)-alkoxy groups, halo(C₁-C₆)alkoxy groups, (C₁-C₆)alkylthio groups, halo(C₁-C₆)alkylthio groups, (C₂-C₆)alkenyl groups, (C₂-C₆)alkynyl groups, and (C₁-C₆)alkoxycarbonyl(C₁-C₆)alkyloxy groups 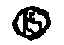 an unsubstituted phenyl(C₁-C₆)alkyl group, 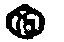 a substituted phenyl(C₁-C₆)alkyl group having on the ring one or more substituents which may be the same or different and are selected from the group consisting of halogen atoms, nitro groups cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, 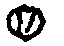 a diphenyl(C₁-C₆)alkyl group, 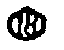 a phenoxy(C₁-C₆)alkyl group, 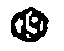 a group represented by the formula:
-B-N(R¹³)R¹⁴
{wherein B is a (C₁-C₆)alkylene group which may be substituted by a (C₁-C₆)alkyl group,
and R¹³ and R¹⁴, which may be the same or different, are hydrogen atoms; formyl groups; (C₁-C₁₂)alkyl groups; (C₂-C₁₂)alkenyl groups; (C₂-C₁₂)alkynyl groups; (C₂-C₁₂)-alkylcarbonyl groups; unsubstituted phenyl groups; substituted phenyl groups having one or more substituents which may be the same or different and are selected from the group consisting of hydrogen atom, halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups; phenylcarbonyl groups; unsubstituted 1,2,3-thiadiazol-5-yl-carbonyl groups; or substituted 1,2,3-thiadiazol-5-yl-carbonyl groups having a halogen atom or a (C₁-C₆)alkyl group as the substituent; R¹³ and R¹⁴ being able to be taken together to represent a (C₄-C₅)alkylene group which may contain, between adjacent carbon atoms of the carbon chain, an oxygen atom, a sulfur atom or
N-R¹¹
(wherein R¹¹ is as defined above)}, 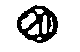 a group represented by the formula:
-C(R¹⁵)=C(R¹⁶)-R¹⁷
(wherein R¹⁵ is a hydrogen atom or a (C₁-C₆)alkyl group, R¹⁶ is a hydrogen atom, a halogen atom or a (C₁-C₆)alkyl group, and R¹⁷ is a nitro group, a cyano group, a (C₁-C₆)alkylcarbonyl group, a (C₁-C₆)alkoxycarbonyl group, a phenylcarbonyl group, or a substituted aminocarbonyl group having one or more substituents which may be the same or different and are selected from the group consisting of hydrogen atom, (C₁-C₁₂)alkyl groups, unsubstituted phenyl group, and substituted phenyl groups having one or more substituents which may be the same or different and are selected from the group consisting of halogen atoms, cyano group, nitro group, (C₁-C₆)alkyl groups and (C₁-C₆)alkoxy groups, R¹⁵ and R¹⁷ being able to be taken together to represent a (C₃-C₆)alkylene group which may be substituted by one or more (C₁-C₆)alkyl group and/or an oxo group), 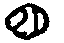 a group represented by the formula: 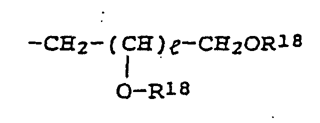 (wherein two R¹⁸'s, which may be the same or different, are hydrogen atoms, (C₁-C₆)alkylcarbonyl groups, phenylcarbonyl groups, unsubstituted 1,2,3-thiadiazol-5-yl-carbonyl groups, or substituted 1,2,3-thiadiazol-5-yl-carbonyl groups having a halogen atom or a (C₁-C₆)-alkyl group as the substituent, and ℓ is zero or an integer of 1 to 12), 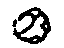 tri(C₁-C₆)alkylsilyl(C₁-C₆)alkyl groups, or 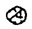 1,2,3-thiadiazol-5-yl-carbonyloxy(C₁-C₁₂)alkyl groups having on the ring a halogen atom or (C₁-C₆)alkyl group as the substituent],

2. The use according to claim 1, wherein R¹ is ① a hydrogen atom, ② a (C₁-C₁₂)alkyl group, ③ a halo(C₁-C₁₂)alkyl group, ④ a (C₂-C₁₂)alkenyl group, ⑤ a halo(C₂-C₁₂)alkenyl group, ⑥ a (C₂-C₁₂)alkynyl group, ⑦ a halo(C₂-C₁₂)alkynyl group, ⑧ a (C₃-C₆)cycloalkyl group, ⑨ an unsubstituted phenyl group, or 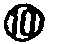 a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, (C₁-C₆)alkylthio groups, halo(C₁-C₆)alkylthio groups, (C₂-C₆)alkenyl groups and (C₂-C₆)alkynyl groups, and
R² is a group represented by the formula:
-X-R¹²
[wherein X is an oxygen atom, and R¹²
is ① a hydrogen atom, ② a (C₁-C₁₂)alkyl group, ③ a halo(C₁-C₁₂)alkyl group, ④ a (C₂-C₂₀)alkenyl group, ⑤ a halo(C₂-C₂₀)alkenyl group, ⑥ a (C₂-C₁₂)alkynyl group, ⑦ a halo(C₂-C₁₂)alkynyl group, ⑧ a hydroxy(C₁-C₆)alkyl group, ⑨ a (C₁-C₁₂)alkoxy(C₁-C₁₂)alkyl group, 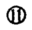 a (C₃-C₆)cycloalkyl group, 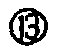 an unsubstituted phenyl group, 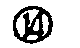 a substituted phenyl group having one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, halo(C₁-C₆)alkoxy groups, and (C₁-C₆)alkoxycarbonyl(C₁-C₆)alkyloxy groups, 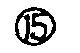 an unsubstituted phenyl(C₁-C₆)alkyl group, 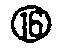 a substituted phenyl(C₁-C₆)alkyl group having on the ring one or more substituents which may be the same or different and are selected from the group consisting of halogen atoms, nitro group, cyano group, (C₁-C₆)alkyl groups, halo(C₁-C₆)alkyl groups, (C₁-C₆)alkoxy groups, and halo(C₁-C₆)alkoxy groups, 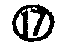 a diphenyl(C₁-C₆)alkyl group, 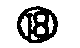 a phenoxy(C₁-C₆)alkyl group, 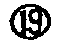 a group represented by the formula:
-B-N(R¹³)R¹⁴
{wherein B is a (C₁-C₆)alkylene group which may be substituted by a (C₁-C₆)alkyl group,
and R¹³ and R¹⁴, which may be the same or different, are hydrogen atoms; formyl groups; (C₁-C₁₂)alkyl groups; (C₂-C₁₂)alkenyl groups; (C₂-C₁₂)alkynyl groups; (C₁-C₁₂)alkylcarbonyl groups; unsubstituted phenyl groups; substituted phenyl groups having one or more substituents which may be the same or different and are selected from the group consisting of hydrogen atom, halogen atoms, nitro group, cyano group, (C₁-C₆)alkyl groups, halo(C₁-C₆)alkyl groups, (C₁-C₆)alkoxy groups, and halo(C₁-C₆)alkoxy groups; phenylcarbonyl groups; unsubstituted 1,2,3-thiadiazol-5-yl-carbonyl groups; or substituted 1,2,3-thiadiazol-5-yl-carbonyl groups having a halogen atom or a (C₁-C₆)alkyl group as the substituent; 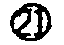 a group represented by the formula: 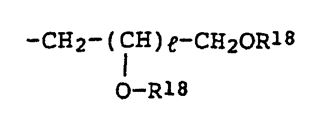 (wherein two R¹⁸'s, which may be the same or different, are hydrogen atoms, (C₁-C₆)alkylcarbonyl groups, phenylcarbonyl groups, unsubstituted 1,2,3-thiadiazol-5-yl-carbonyl groups, or substituted 1,2,3-thiadiazol-5-yl-carbonyl groups having a halogen atom or a (C₁-C₆)-alkyl group as the substituent, and ℓ is zero or an integer of 1 to 12), or 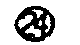 1,2,3-thiadiazol-5-yl-carbonyloxy(C₁-C₆)alkyl groups having on the ring a halogen atom or (C₁-C₆)alkyl group as the substituent].

3. A method for controlling agricultural and horticultural diseases which comprises applying the composition for controlling agricultural and horticultural diseases as defined in claim 1 or 2 in a dosage of 0.1 to 10 kg (in terms of the active ingredient of the disease controller) per 1000 m² ( 10 ares ) for protecting useful crop against the diseases.

4. Use of a 1,2,3-thiadiazole derivative represented by the general formula (I), or a salt thereof 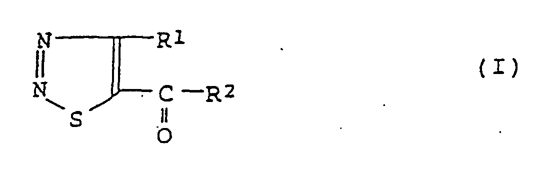 wherein R¹ and R² are as defined in claim 1 for controlling agricultural and horticultural diseases, which damage crops, fruit trees, vegetables, flowers and ornamental plants.

## Patentansprüche

1. Verwendung einer Zusammensetzung, die einen inerten Träger und eine aktive Menge eines 1,2,3-Thiadiazol-Derivats der allgemeinen Formel (I) oder ein Salz davon enthält, zur Bekämpfung von Krankheiten in der Landwirtschaft und im Gartenbau, die Feldfrüchte, Obstbäume, Gemüse, Blumen und Zierpflanzen schädigen: 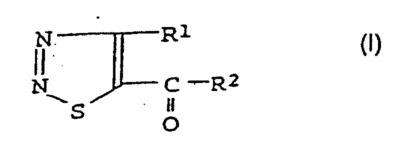 worin R¹ **(1)** ein Wasserstoffatom, **(2)** eine (C₁-C₁₂)Alkylgruppe, **(3)** eine Halo(C₁-C₁₂)alkylgruppe, **(4)** eine (C₂-C₁₂)Alkenylgruppe, **(5)** eine Halo(C₂-C₁₂)alkenylgruppe, **(6)** eine (C₂-C₁₂)Alkynylgruppe, **(7)** eine Halo(C₂-C₁₂)alkynylgruppe, **(8)** eine (C₃-C₆)cycloalkylgruppe, (9) eine unsubstituierte Phenylgruppe, **(10)** eine substituierte Phenylgruppe mit einem oder mehreren Substituenten, die gleich oder verschieden sein können und gewählt werden aus der Gruppe bestehend aus Halogenatomen, Nitrogruppen, Cyanogruppen, (C₁-C₆)alkylgruppen, Halo(C₁-C₆)Alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)alkynylgruppen, **(11)** eine Gruppe der Formel: 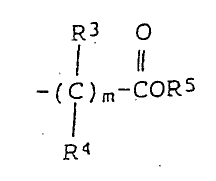 (worin R³ und R⁴, die gleich oder verschieden sein können, Wasserstoffatome, (C₁-C₁₂)Alkylgruppen oder Halo(C₁-C₁₂)alkylgruppen sind, m Null oder eine ganze Zahl von 1 bis 6 ist; und R⁵ ein Wasserstoffatom; eine (C₁-C₁₂)Alkylgruppe; eine Halo(C₁-C₁₂)alkylgruppe; eine (C₂-C₁₂)alkenylgruppe; eine (C₂-C₁₂)Alkynylgruppe; eine unsubstituierte Phenylgruppe; eine substituierte Phenylgruppe mit einem oder mehreren Substituenten, die gleich oder verschieden sein können und gewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)alkenylgruppen und (C₂-C₆)alkynylgruppen; eine unsubstituierte Phenyl(C₁-C₆)Alkylgruppe; oder eine substituierte Phenyl (C₁-C₆)Alkylgruppe mit einem oder mehreren Substituenten am Ring, die gleich oder verschieden sein können und abgewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)Alkynylgruppen, **(12)** eine Gruppe der Formel: 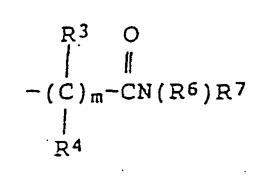 (worin R³, R⁴ und m wie oben definiert sind, und R⁶ und R⁷, die gleich oder verschieden sein können, Wasserstoffatome; (C₁-C₁₂)Alkylgruppen; Halo(C₁-C₁₂)alkylgruppen; (C₂-C₁₂)Alkenylgruppen; (C₂-C₁₂)Alkynylgruppen; (C₁-C₁₂)Alkoxy(C₁-C₆)alkylgruppen; (C₁-C₁₂)Alkylthio(C₁-C₆)alkylgruppen; Cyano(C₁-C₁₂)alkylgruppen; unsubstituierte Phenylgruppen; substituierte Phenylgruppen mit einem oder mehreren Substituenten, die gleich oder verschieden sein können und gewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)Alkynylgruppen; unsubstituierte Phenyl(C₁-C₆)Alkylgruppen; oder substituierte Phenyl(C₁-C₆)alkylgruppen mit einem oder mehreren Substituenten am Ring, die gleich oder verschieden sein können und abgewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)Alkynylgruppen, sind; R⁶ und R⁷ miteinander verbunden sein können, um eine (C₄-C₆)alkylengruppe zu bilden, die ein Sauerstoffatom zwischen benachbarten Kohlenstoffatomen der Kohlenstoffkette enthalten kann), **(13)** eine Gruppe der Formel: 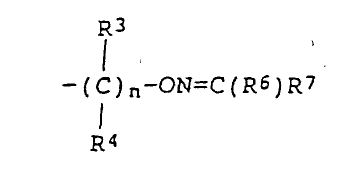 (worin R³, R⁴, R⁶ und R⁷ wie oben definiert sind und n eine ganze Zahl von 1 bis 6 ist), oder **(14)** eine Gruppe der Formel: 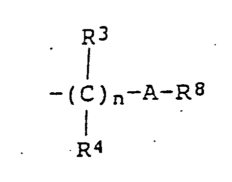 [worin R³, R⁴ und n wie oben definiert sind, A
-O- , 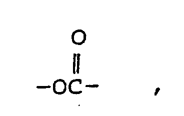 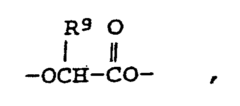
(worin R⁹ ein Wasserstoffatom, eine (C₁-C₁₂)Alkylgruppe oder eine Halo(C₁-C₁₂)alkylgruppe ist),
-S- ,
-SO- ,
-SO₂- , 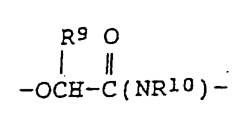
{worin R⁹ wie oben definiert ist und R¹⁰ ein Wasserstoffatom, eine (C₁-C₁₂)Alkylgruppe oder eine Halo(C₁-C₁₂)alkylgruppe ist, R⁹ und R¹⁰ miteinander verbunden sein können um eine (C₄-C₆)Alkylengruppe zu bilden, die zwischen benachbarten Kohlenstoffatomen der Kohlenstoffkette ein Sauerstoffatom, ein Schwefelatom oder
N-R¹¹
enthalten kann,
(worin R¹¹ ein Wasserstoffatom, eine (C₁-C₁₂)Alkylgruppe oder eine Halo(C₁-C₁₂)alkylgruppe ist)}, oder
-N(R¹⁰)-
(worin R¹⁰ wie oben definiert ist), bedeutet, und R⁸ ein Wasserstoffatom; eine (C₁-C₁₂)Alkylgruppe; eine Halo(C₁-C₁₂)alkylgruppe; eine (C₂-C₁₂)Alkenylgruppe; eine (C₂-C₁₂)Alkynylgruppe; eine (C₁-C₁₂)Alkoxy(C₁-C₁₂)alkylgruppe; eine (C₁-C₁₂)Alkylthio(C₁-C₁₂)alkylgruppe; eine Cyano(C₁-C₁₂)alkylgruppe; eine unsubstituierte Phenylgruppe; eine substituierte Phenylgruppe mit einem oder mehreren Substituenten, die gleich oder verschieden sein können und gewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)Alkynylgruppen; eine unsubstituierte Phenyl (C₁-C₆)Alkylgruppe; eine substituierte Phenyl (C₁-C₆)alkylgruppe mit einem oder mehreren Substituenten am Ring, die gleich oder verschieden sein können und abgewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)Alkynylgruppen; ein unsusbtituierter 5- oder 6-gliedriger heterocyclischer Ring mit einem oder mehreren Heteroatomen, die gleich oder verschieden sein können und gewählt werden aus der Gruppe bestehend aus Sauerstoffatomen, Schwefelatomen und Stickstoffatomen; oder ein substituierter 5- oder 6-gliedriger heterocyclischer Ring mit einem oder mehreren Heteroatomen, die gleich oder verschieden sein können und gewählt werden aus der Gruppe bestehend aus Sauerstoffatomen, Schwefelatomen und Stickstoffatomen, wobei der substituierte heterocyclische Ring einen oder mehrere Substituenten aufweist, die gleich oder verschieden sein können und gewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und
(C₂-C₆)Alkynylgruppen, ist] und
R² eine Gruppe der Formel
-X-R¹²
ist [worin X ein Sauerstoffatom ist und R¹² **(1)** ein Wasserstoffatom, **(2)** eine (C₁-C₁₂)Alkylgruppe, **(3)** eine Halo(C₁-C₁₂)alkylgruppe, **(4)** eine (C₂-C₂₀)Alkenylgruppe, **(5)** eine Halo(C₂-C₂₀)alkenylgruppe, **(6)** eine (C₂-C₁₂)Alkynylgruppe, **(7)** eine Halo(C₂-C₁₂)Alkynylgruppe, **(8)** eine Hydroxy(C₁-C₆)alkylgruppe, **(9)** eine (C₁-C₁₂)Alkoxy(C₁-C₁₂)alkylgruppe, **(10)** eine (C₁-C₁₂)Alkylthio(C₁-C₁₂)alkylgruppe, **(11)** eine (C₃-C₆)Cycloalkylgruppe, **(13)** eine unsubstituierte Phenylgruppe, **(14)** eine substituierte Phenylgruppe mit einem oder mehreren Substituenten, die gleich oder verschieden sein können und gewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen, (C₂-C₆)Alkynylgruppen und (C₁-C₆)Alkoxycarbonyl(C₁-C₆)alkyloxygruppen, **(15)** eine unsubstituierte Phenyl (C₁-C₆)alkylgruppe, **(16)** eine substituierte Phenyl(C₁-C₆)alkylgruppe mit einem oder mehreren Substituenten am Ring, die gleich oder verschieden sein können und gewählt werden aus der Gruppe bestehend aus Halogenatomen, Nitrogruppen, Cyanogruppen, (C₁-C₆)Alkylgruppen, Halo (C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)Alkynylgruppen, **(17)** eine Diphenyl(C₁-C₆)alkylgruppe, **(18)** eine Phenoxy(C₁-C₆)alkylgruppe, **(19)** eine Gruppe der Formel:
-B-N(R¹³)R¹⁴
{worin B eine (C₁-C₆)Alkylengruppe ist, die substituiert sein kann durch eine (C₁-C₆)Alkylgruppe, und R¹³ und R¹⁴, die gleich oder verschieden sein können, Wasserstoffatome; Formylgruppen; (C₁-C₁₂)Alkylgruppen; (C₂-C₁₂)Alkenylgruppen; (C₂-C₁₂)Alkynylgruppen; (C₂-C₁₂)Alkylcarbonylgruppen; unsubstituierte Phenylgruppen; substituierte Phenylgruppen mit einem oder mehreren Substituenten, die gleich oder verschieden sein können und gewählt werden aus der Gruppe bestehend aus Wasserstoffatomen, Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)Alkynylgruppen; Phenylcarbonylgruppen; unsubstituierte 1,2,3-Thiadiazol-5-ylcarbonylgruppen; oder substituierte 1,2,3-Thiadiazol-5-yl-carbonylgruppen mit einem Haolgenatom oder einer (C₁-C₆)Alkylgruppe als Substituent sind; oder R¹³ und R¹⁴ miteinander verbunden sein können um eine (C₄-C₅)Alkylengruppe zu bilden, die zwischen benachbarten Kohlenstoffatomen der Kohlenstoffkette ein Sauerstoffatom, eine Schwefelatom oder
N-R¹¹
enthalten können (worin R¹¹ wie oben definiert ist)}, **(20)** eine Gruppe der Formel:
-C(R¹⁵)=C(R¹⁶)-R¹⁷
(worin R¹⁵ ein Wasserstoffatom oder eine (C₁-C₆)Alkylgruppe ist, R¹⁶ ein Wasserstoffatom, ein Halogenatom oder eine (C₁-C₆)Alkylgruppe ist und R¹⁷ eine Nitrogruppe, eine Cyanogruppe, eine (C₁-C₆)Alkylcarbonylgruppe, eine (C₁-C₆)Alkoxycarbonylgruppe, eine Phenylcarbonylgruppe oder eine substituierte Aminocarbonylgruppe mit einem oder mehreren Substituenten ist, die gleich oder verschieden sein können und gewählt werden aus der Gruppe bestehend aus einem Wasserstoffatom, (C₁-C₁₂)Alkylgruppen, unsubstituierten Phenylgruppen und substituierte Phenylgruppen mit einem oder mehreren Substituenten, die gleich oder verschieden sein können und gewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, R¹⁵ und R¹⁷ miteinander verbunden sein können um eine (C₃-C₆)Alkylengruppe zu bilden, die durch einen oder mehrere (C₁-C₆)Alkylgruppen und/oder eine Oxogruppe substituiert sein kann) **(21)** eine Gruppe der Formel: 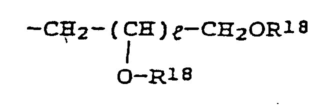 (worin die beiden Gruppen R¹⁸, die gleich oder verschieden sein können, Wasserstoffatome, (C₁-C₆)Alkylcarbonylgruppen, Phenylcarbonylgruppen, unsubstituierte 1,2,3-Thiadiazol-5-yl-carbonylgruppen, oder substituierte 1,2,3-Thiadiazol-5-yl-carbonylgruppen mit einem Halogenatom oder einer (C₁-C₆)Alkylgruppe als Substituent sind, und ℓ Null oder eine ganze zahl von 1 bis 12 ist), **(23)** Tri(C₁-C₆)alkylsilyl(C₁-C₆)alkylgruppen, oder **(24)** 1,2,3-Thiadiazol-5-yl-carbonyloxy(C₁-C₁₂)alkylgruppen mit einem Halogenatom oder (C₁-C₆)Alkylgruppe als Substituent am Ring].

2. Verwendung nach Anspruch 1, worin R¹ **(1)** ein Wasserstoffatom, **(2)** eine (C₁-C₁₂)Alkylgruppe, **(3)** eine Halo(C₁-C₁₂)alkylgruppe, **(4)** eine (C₂-C₁₂)Alkenylgruppe, **(5)** eine Halo(C₂-C₁₂)alkenylgruppe, **(6)** eine (C₂-C₁₂)Alkynylgruppe, **(7)** eine Halo(C₂-C₁₂)alkynylgruppe, **(8)** eine (C₃-C₆)Cycloalkylgruppe, **(9)** eine unsubstituierte Phenylgruppe, **(10)** eine substituierte Phenylgruppe mit einem oder mehreren Substituenten, die gleich oder verschieden sein können und gewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, (C₁-C₆)Alkylthiogruppen, Halo(C₁-C₆)alkylthiogruppen, (C₂-C₆)Alkenylgruppen und (C₂-C₆)Alkynylgruppen, und R² eine Gruppe der Formel
-X-R¹²
ist [worin X ein Sauerstoffatom ist und R¹² **(1)** ein Wasserstoffatom, **(2)** eine (C₁-C₁₂)Alkylgruppe, **(3)** eine Halo(C₁-C₁₂)alkylgruppe, **(4)** eine (C₂-C₂₀)Alkenylgruppe, **(5)** eine Halo(C₂-C₂₀)alkenylgruppe, (6) eine (C₂-C₁₂)Alkynylgruppe, **(7)** eine Halo(C₂-C₁₂)alkynylgruppe, **(8)** eine Hydroxy(C₁-C₆)alkylgruppe, **(9)** eine (C₁-C₁₂)Alkoxy(C₁-C₁₂)alkylgruppe, **(11)** eine (C₃-C₆)Cycloalkylgruppe, **(13)** eine unsubstituierte Phenylgruppe, **(14)** eine substituierte Phenylgruppe mit einem oder mehreren Substituenten, die gleich oder verschieden sein können und gewählt werden aus der Gruppe bestehend aus Halogenatomen, Nitrogruppen, Cyanogruppen, (C₁-C₆)Alkylgruppen, Halo(C₁-C₆)alkylgruppen, (C₁-C₆)Alkoxygruppen, Halo(C₁-C₆)alkoxygruppen, und (C₁-C₆)Alkoxycarbonyl(C₁-C₆)alkyloxygruppen, **(15)** eine unsubstituierte Phenyl (C₁-C₆)alkylgruppe, **(16)** eine substituierte Phenyl (C₁-C₆)alkylgruppe mit einem oder mehreren Substituenten am Ring, die gleich oder verschieden sein können und gewählt werden aus der Gruppe bestehend aus Halogenatomen, Nitrogruppen, Cyanogruppen, (C₁-C₆)Alkylgruppen, halo(C₁-C₆)alkylgruppen, (C₁-C₆)Alkoxygruppen und Halo(C₁-C₆)alkoxygruppen, **(17)** eine Diphenyl(C₁-C₆)alkylgruppe, **(18)** eine Phenoxy(C₁-C₆)alkylgruppe, **(19)** eine Gruppe der Formel:
-B-N(R¹³)R¹⁴
{worin B eine (C₁-C₆)Alkylengruppe ist, die mit einer (C₁-C₆)Alkylgruppe substituiert sein kann, und R¹³ und R¹⁴, die gleich oder verschieden sein können, Wasserstoffatome; Formylgruppen; (C₁-C₁₂)Alkylgruppen; (C₂-C₁₂)Alkenylgruppen; (C₂-C₁₂)Alkynylgruppen; (C₂-C₁₂)Alkylcarbonylgruppen; unsubstituierte Phenylgruppen; substituierte Phenylgruppen mit einem oder mehreren Substituenten, die gleich oder verschieden sein können und gewählt werden aus der Gruppe bestehend aus Wasserstoffatomen, Halogenatomen, Nitrogruppen, Cyanogruppen, (C₁-C₆)Alkylgruppen, Halo(C₁-C₆)alkylgruppen, (C₁-C₆)Alkoxygruppen und Halo(C₁-C₆)alkoxygruppen; Phenylcarbonylgruppen; unsubstituierte 1,2,3-Thiadiazol-5-yl-carbonylgruppen; oder substituierte 1,2,3-Thiadiazol-5-ylcarbonylgruppen mit einem Haolgenatom oder einer (C₁-C₆)Alkylgruppe als Substituent sind}; **(21)** eine Gruppe der Formel: 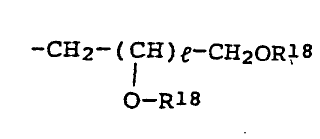 (worin die beiden Gruppen R¹⁸, die gleich oder verschieden sein können, Wasserstoffatome, (C₁-C₆)Alkylcarbonylgruppen, Phenylcarbonylgruppen, unsubstituierte 1,2,3-Thiadiazol-5-yl-carbonylgruppen, oder substituierte 1,2,3-Thiadiazol-5-yl-carbonylgruppen mit einem Halogenatom oder einer (C₁-C₆)alkylgruppe als Substituent sind, und ℓ Null oder eine ganze zahl von 1 bis 12 ist), oder **(24)** 1,2,3-Thiadiazol-5-yl-carbonyloxy(C₁-C₁₂)alkylgruppen mit einem Halogenatom oder (C₁-C₆)Alkylgruppe als Substituent am Ring].

3. Verfahren zur Bekämpfung von Krankheiten in der Landwirtschaft und im Gartenbau umfassend das Anwenden der Zusammensetzung zur Bekämpfung von Krankheiten in der Landwirtschaft und im Gartenbau, wie in Anspruch 1 oder 2 definiert, in einer Dosierung von 0,1 bis 10 kg (bezogen auf den aktiven Inhaltsstoff des Krankheitsbekämpfungsmittels) pro 1000 m² (10 Ar) zum Schutz von nutzbaren Feldfrüchten gegen die Krankheiten.

4. Verwendung eines 1,2,3-Thiadiazolderivates der allgemeinen Formel (I) oder eines Salzes davon 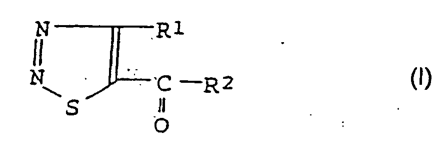 worin R¹ und R² wie in Anspruch 1 definiert sind, zur Bekämpfung von Krankheiten in der Landwirtschaft und im Gartenbau, die Feldfrüchte, Obstbäume, Gemüse, Blumen und Zierpflanzen schädigen.

## Revendications

1. Utilisation d'une composition qui comprend un support inerte et une quantité active d'un dérivé de 1,2,3-thiadiazole représenté par la formule générale (I), ou un sel de celui-ci afin de lutter contre les maladies dans les domaines de l'agriculture et de l'horticulture qui abîment les cultures, les arbres fruitiers, les légumes, les fleurs, et les plantes de décoration : 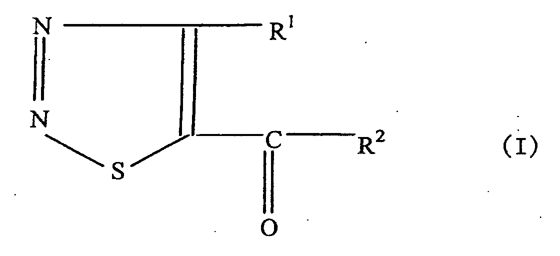 dans laquelle R¹ représente (1) un atome d'hydrogène, (2) un groupe alkyle en C₁-C₁₂, (3) un groupe halogéno(alkyle en C₁-C₁₂), (4) un groupe alcényle en C₂-C₁₂, (5) un groupe halogéno(alcényle en C₂-C₁₂), (6) un groupe alcynyle en C₂-C₁₂, (7) un groupe halogéno(alcynyle en C₂-C₁₂), (8) un groupe cycloalkyle en C₃-C₆, (9) un groupe phényle non substitué, (10) un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆, et les groupes alcynyle en C₂-C₆, (11) un groupe représenté par la formule : 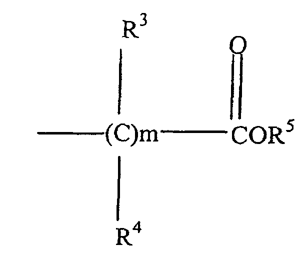 (dans laquelle R³ et R⁴, qui peuvent être identiques ou différents, représentent des atomes d'hydrogène, des groupes alkyle en C₁-C₁₂ ou des groupes halogéno(alkyle en C₁-C₁₂), m vaut zéro ou est un nombre entier de 1 à 6, et R⁵ représente un atome d'hydrogène ; un groupe alkyle en C₁-C₁₂ ; un groupe halogéno(alkyle en C₁-C₁₂) ; un groupe alcényle en C₂-C₁₂ ; un groupe alcynyle en C₂-C₁₂ ; un groupe phényle non substitué ; un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆ et les groupes alcynyle en C₂-C₆ ; un groupe phényl(alkyle en C₁-C₆) non substitué ; ou un groupe phényl(alkyle en C₁-C₆) substitué ayant sur le noyau un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆ et les groupes alcynyle en C₂-C₆), (12) un groupe représenté par la formule : 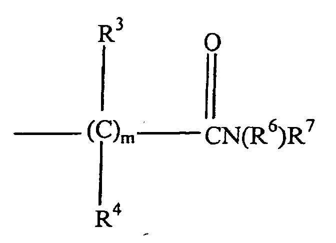 (dans laquelle R³, R⁴ et m sont comme définis ci-dessus, et R⁶ et R⁷, qui peuvent être identiques ou différents, représentent des atomes d'hydrogène ; des groupes alkyle en C₁-C₁₂ ; des groupes halogéno(alkyle en C₁-C₁₂) ; des groupes alcényle en C₂-C₁₂ ; des groupes alcynyle en C₂-C₁₂ ; des groupes (alcoxy en C₁-C₁₂) (alkyle en C₁-C₆) ; des groupes (alkyle en C₁-C₁₂)thio(alkyle en C₁-C₆) ; des groupes cyano(alkyle en C₁-C₁₂) ; des groupes phényle non substitués ; des groupes phényle substitués ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆ et les groupes alcynyle en C₂-C₆ ; des groupes phényl(alkyle en C₁-C₆) non substitués ; ou des groupes phényl(alkyle en C₁-C₆) substitués ayant sur le cycle un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆ et les groupes alcynyle en C₂-C₆ ; R⁶ et R⁷ pouvant être pris conjointement afin de représenter un groupe alkylène en C₄-C₆ qui peut contenir un atome d'oxygène entre des atomes de carbone adjacents de la chaîne carbonée), (13) un groupe représenté par la formule : 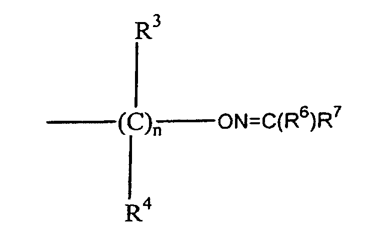 (dans laquelle R³, R⁴, R⁶ et R⁷ sont comme définis ci-dessus, et n est un nombre entier de 1 à 6) , ou (14) un groupe représenté par la formule : 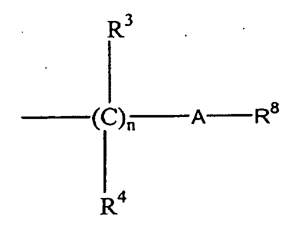 [(dans laquelle R³, R⁴ et n sont comme définis ci-dessus, A est
-O- , 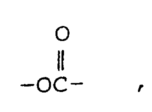 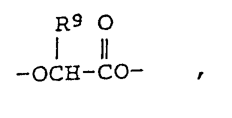
(dans lequel R⁹ représente un atome d'hydrogène, un groupe alkyle en C₁-C₁₂, un groupe halogéno(alkyle en C₁-C₁₂)),
-S- ,
-SO- ,
-SO₂- , 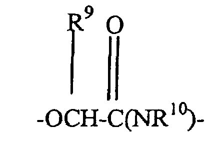
(dans lequel R⁹ est comme défini ci-dessus, et R¹⁰ représente un atome d'hydrogène, un groupe alkyle en C₁-C₁₂, ou un groupe halogéno(alkyle en C₁-C₁₂), R⁹ et R¹⁰ pouvant être pris conjointement afin de représenter un groupe alkylène en C₄-C₆ qui peut contenir, entre des atomes de carbone adjacents de la chaîne carbonée, un atome d'oxygène, un atome de soufre ou
N-R¹¹
(dans lequel R¹¹ représente un atome d'hydrogène, un groupe alkyle en C₁-C₁₂ ou un groupe halogéno(alkyle en C₁-C₁₂))}, ou
-N(R¹⁰)-
(dans lequel R¹⁰ est comme défini ci-dessus), et R⁸ représente un atome d'hydrogène ; un groupe alkyle en C₁-C₁₂ ; un groupe halogéno(alkyle en C₁-C₁₂) ; un groupe alcényle en C₂-C₁₂ ; un groupe alcynyle en C₂-C₁₂ ; un groupe (alcoxy en C₁-C₁₂) (alkyle en C₁-C₁₂) ; un groupe (alkyle en C₁-C₁₂)thio(alkyle en C₁-C₁₂) ; un groupe cyano(alkyle en C₁-C₁₂) ; un groupe phényle non substitué ; un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆ et les groupes alcynyle en C₂-C₆ ; un groupe phényl(alkyle en C₁-C₆) non substitué ; un groupe phényl(alkyle en C₁-C₆) substitué ayant sur le cycle un ou plusieurs substituants pouvant être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆ et les groupes alcynyle en C₂-C₆ ; un cycle hétérocyclique non substitué à 5 ou 6 chaînons ayant un ou plusieurs hétéroatomes qui peuvent être identiques ou différents et sont choisis dans le groupe constitué par un atome d'oxygène, un atome de soufre et un atome d'azote ; ou un cycle hétérocyclique substitué à 5 ou 6 chaînons ayant un ou plusieurs hétéroatomes qui peuvent être identiques ou différents et sont choisis dans le groupe constitué par un atome d'oxygène, un atome de soufre et un atome d'azote, ledit noyau hétérocyclique substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆ et les groupes alcynyle en C₂-C₆], et R² est un groupe représenté par la formule :
-X-R¹²
[dans laquelle X représente un atome d'oxygène, et R¹² représente (1) un atome d'hydrogène, (2) un groupe alkyle en C₁-C₁₂, (3) un groupe halogéno(alkyle en C₁-C₁₂), (4) un groupe alcényle en C₂-C₂₀, (5) un groupe halogéno(alcényle en C₂-C₂₀), (6) un groupe alcynyle en C₂-C₁₂, (7) un groupe halogéno(alcynyle en C₂-C₁₂), (8) un groupe hydroxy(alkyle en C₁-C₆), (9) un groupe (alcoxy en C₁-C₁₂)(alkyle en C₁-C₁₂), (10) un groupe (alkyle en C₁-C₁₂)thio(alkyle en C₁-C₁₂), (11) un groupe cycloalkyle en C₃-C₆, (13) un groupe phényle non substitué, (14) un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, -les groupes halogéno(alcoxy en C₁-C₆), les groupes (alkyle en C₁-C₆)thio, les groupes halogéno(alkyle en C₁-C₆)thio, les groupes alcényle en C₂-C₆, les groupes alcynyle en C₂-C₆, et les groupes (alcoxy en C₁-C₆)carbonyl(alcoxy en C₁-C₆), (15) un groupe phényl(alkyle en C₁-C₆) non substitué, (16) un groupe phényl(alkyle en C₁-C₆) substitué ayant sur le noyau un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆ et les groupes alcynyle en C₂-C₆, (17) un groupe diphényl(alkyle en C₁-C₆), (18) un groupe phénoxy(alkyle en C₁-C₆), (19) un groupe représenté par la formule :
-B-N(R¹³)R¹⁴
{dans laquelle B représente un groupe alkylène en C₁-C₆ qui peut être substitué par un groupe alkyle en C₁-C₆, et R¹³ et R¹⁴, qui peuvent être identiques ou différents, représentent des atomes d'hydrogène ; des groupes formyle ; des groupes alkyle en C₁-C₁₂ ; des groupes alcényle en C₂-C₁₂ ; des groupes alcynyle en C₂-C₁₂ ; des groupes (alkyle en C₂-C₁₂)carbonyle ; des groupes phényle non substitués ; des groupes phényle substitués ayant un ou plusieurs substituants qui peuvent être identiques ou différents et sont choisis dans le groupe constitué par un atome d'hydrogène, les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₂-C₆)thio, les groupes alcényle en C₂-C₆, et les groupes alcynyle en C₂-C₆ ; des groupes phénylcarbonyle ; des groupes 1,2,3-thiadiazol-5-yl-carbonyle non substitués ; ou des groupes 1,2,3-thiadiazol-5-yl-carbonyle ayant un atome d'halogène ou un groupe alkyle en C₁-C₆ en tant que substituant ; R¹³ et R¹⁴ pouvant être pris conjointement afin de représenter un groupe alkylène en C₄-C₅ qui peut contenir, entre des atomes de carbone adjacents de la chaîne carbonée, un atome d'oxygène, un atome de soufre ou
N-R¹¹
(dans lequel R¹¹ est comme défini ci-dessus)}, (20) un groupe représenté par la formule :
-C(R¹⁵)=C(R¹⁶)-R¹⁷
(dans laquelle R¹⁵ représente un atome d'hydrogène ou un groupe alkyle en C₁-C₆, R¹⁶ représente un atome d'hydrogène, un atome d'halogène ou un groupe alkyle en C₁-C₆, et R¹⁷ représente un groupe nitro, un groupe cyano, un groupe (alkyle en C₁-C₆)carbonyle, un groupe (alcoxy en C₁-C₆)carbonyle, un groupe phénylcarbonyle, ou un groupe aminocarbonyle substitué ayant un ou plusieurs substituants qui peuvent être identiques ou différents et sont choisis dans le groupe constitué par un atome d'hydrogène, les groupes alkyle en C₁-C₁₂, un groupe phényle non substitué, et les groupes phényle substitués ayant un ou plusieurs substituants qui peuvent être identiques ou différents et sont choisis dans le groupe constitué par les atomes d'halogène, un groupe cyano, un groupe nitro, les groupes alkyle en C₁-C₆, et les groupes alcoxy en C₁-C₆, R¹⁵ et R¹⁷ pouvant être pris conjointement afin de représenter un groupe alkylène en C₃-C₆ qui peut être substitué par un ou plusieurs groupes alkyle en C₁-C₆ et/ou un groupe oxo), (21) un groupe représenté par la formule : 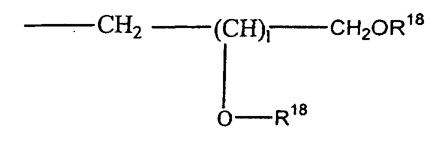 (dans laquelle les deux groupes R¹⁸, lesquels peuvent être identiques ou différents, représentent des atomes d'hydrogène, des groupes (alkyle en C₁-C₆)carbonyle ; des groupes phénylcarbonyle, des groupes 1,2,3-thiadiazol-5-yl-carbonyle non substitués, ou des groupes 1,2,3-thiadiazol-5-yl-carbonyle substitués ayant un atome d'halogène ou un groupe alkyle en C₁-C₆ en tant que substituant, et 1 vaut zéro ou est un nombre entier allant de 1 à 12), (23) des groupes tri(alkyle en C₁-C₆)silyl(alkyle en C₁-C₆), ou (24) des groupes 1,2,3-thiadiazol-5-yl-carbonyloxy(alkyle en C₁-C₁₂) ayant sur le cycle un atome d'halogène ou un groupe alkyle en C₁-C₆ en tant que substituant].

2. Utilisation selon la revendication 1, dans laquelle R¹ représente (1) un atome d'hydrogène, (2) un groupe alkyle en C₁-C₁₂, (3) un groupe halogéno(alkyle en C₁-C₁₂), (4) un groupe alcényle en C₂-C₁₂, (5) un groupe halogéno(alcényle en C₂-C₁₂), (6) un groupe alcynyle en C₂-C₁₂, (7) un groupe halogéno(alcynyle en C₂-C₁₂), (8) un groupe cycloalkyle en C₃-C₆, (9) un groupe phényle non substitué, ou (10) un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), les groupes (alkyle en C₁-C₆)thio, les groupes halogéno(alkyle en C₁-C₆)thio, les groupes alcényle en C₂-C₆, et les groupes alcynyle en C₂-C₆, et
R² est un groupe représenté par la formule :
-X-R¹²
[dans laquelle X représente un atome d'oxygène, et R¹² représente (1) un atome d'hydrogène, (2) un groupe alkyle en C₁-C₁₂, (3) un groupe halogéno(alkyle en C₁-C₁₂), (4) un groupe alcényle en C₂-C₂₀, (5) un groupe halogéno(alcényle en C₂-C₂₀), (6) un groupe alcynyle en C₂-C₁₂, (7) un groupe halogéno(alcynyle en C₂-C₁₂), (8) un groupe hydroxy(alkyle en C₁-C₆), (9) un groupe (alcoxy en C₁-C₁₂) (alkyle en C₁-C₁₂), (11) un groupe cycloalkyle en C₃-C₆, (13) un groupe phényle non substitué, (14) un groupe phényle substitué ayant un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), et les groupes (alcoxy en C₁-C₆)carbonyl(alcoxy en C₁-C₆), (15) un groupe phényl(alkyle en C₁-C₆) non substitué, (16) un groupe phényl(alkyle en C₁-C₆) substitué ayant sur le cycle un ou plusieurs substituants qui peuvent être identiques ou différents et sont choisis dans le groupe constitué par les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆), (17) un groupe diphényl(alkyle en C₁-C₆), (18) un groupe phénoxy(alkyle en C₁-C₆), (19) un groupe représenté par la formule :
-B-N(R¹³)R¹⁴
(dans laquelle B représente un groupe alkylène en C₁-C₆ qui peut être substitué par un groupe alkyle en C₁-C₆, et R¹³ et R¹⁴, qui peuvent être identiques ou différents, représentent des atomes d'hydrogène ; des groupes formyle ; des groupes alkyle en C₁-C₁₂ ; des groupes alcényle en C₂-C₁₂ ; des groupes alcynyle en C₂-C₁₂ ; des groupes (alkyle en C₁-C₁₂)carbonyle ; des groupes phényle non substitués ; des groupes phényle substitués ayant un ou plusieurs substituants qui peuvent être identiques ou différents et sont choisis dans le groupe constitué par un atome d'hydrogène, les atomes d'halogène, un groupe nitro, un groupe cyano, les groupes alkyle en C₁-C₆, les groupes halogéno(alkyle en C₁-C₆), les groupes alcoxy en C₁-C₆, les groupes halogéno(alcoxy en C₁-C₆) ; des groupes phénylcarbonyle ; des groupes 1,2,3-thiadiazol-5-yl-carbonyle non substitués ; ou des groupes 1,2,3-thiadiazol-5-yl-carbonyle substitués ayant un atome d'halogène ou un groupe alkyle en C₁-C₆ en tant que substituant ; (21) un groupe représenté par la formule : 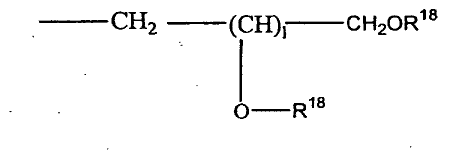 (dans laquelle les deux groupes R¹⁸, lesquels peuvent être identiques ou différents, représentent des atomes d'hydrogène, des groupes (alkyle en C₁-C₆)carbonyle, des groupes phénylcarbonyle, des groupes 1,2,3-thiadiazol-5-yl-carbonyle non substitués, ou des groupes 1,2,3-thiadiazol-5-yl-carbonyle ayant un atome d'halogène ou un groupe alkyle en C₁-C₆ en tant que substituant, et 1 vaut zéro ou est un nombre entier de 1 à 12), ou (24) des groupes 1,2,3-thiadiazol-5-yl-carbonyloxy(alkyle en C₁-C₆) ayant sur le cycle un atome d'halogène ou un groupe alkyle en C₁-C₆ en tant que substituant].

3. Procédé de lutte contre les maladies dans les domaines de l'agriculture et de l'horticulture qui comprend l'application de la composition destinée à lutter contre les maladies dans les domaines de l'agriculture et de l'horticulture selon la revendication 1 ou 2 dans une dose de 0,1 g à 10 kg (en termes de la substance active de l'agent de lutte contre la maladie) par 1000 m² (10 ares) afin de protéger les cultures utiles contre les maladies.

4. Utilisation d'un dérivé de 1,2,3-thiadiazole représenté par la formule générale (I), ou un sel de celui-ci : 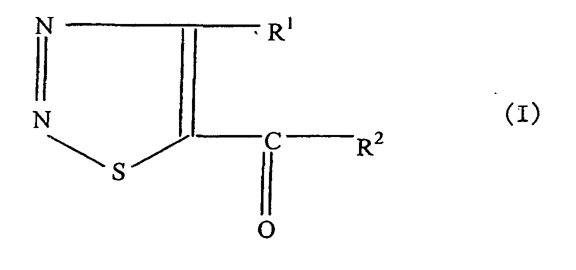 dans laquelle R¹ et R² sont comme définis selon la revendication 1 afin de lutter contre les maladies dans les domaines de l'agriculture et de l'horticulture, qui abîment les cultures, les arbres fruitiers, les légumes, les fleurs, et les plantes de décoration.
